# FASCICULE DE BREVET EUROPEEN

(11) **EP 3 890 678 B1**
(45) Date de publication et mention de la délivrance du brevet: **17.06.2026**
(21) Numéro de dépôt: 19835433.4
(22) Date de dépôt: 25.11.2019
(51) Int. Cl.: A61J 1/10

(54) **RESERVOIR DE LIQUIDE BIOPHARMACEUTIQUE AVEC ORGANE MECANIQUE INCLUANT DES ENSEMBLES DE PIECES TOURNANT ET FIXE**
BIOPHARMAZEUTISCHES FLÜSSIGKEITSRESERVOIR MIT MECHANISCHEM ELEMENT MIT ROTIERENDEN UND STATIONÄREN SÄTZEN VON TEILEN
BIOPHARMACEUTICAL LIQUID RESERVOIR WITH MECHANICAL MEMBER INCLUDING ROTATING AND STATIONARY SETS OF PARTS

(30) Priorité: 06.12.2018 FR 1872448
(43) Date de publication de la demande: 13.10.2021
(73) Titulaire: Sartorius Stedim FMT, 13400 Aubagne (FR)
(72) Inventeur: GIBELIN, Jérémy, 83870 Signes (FR); BOURSEAUX, David, 13190 Allauch (FR); LOISELET, Yanis, 13600 Ceyreste (FR)
(74) Mandataire: Novagraaf International SA
(86) Numéro de dépôt international: PCT/FR2019/000191
(87) Numéro de publication internationale: WO 2020/115372

(56) Documents cités:
- US-A- 5 385 546

## Description

### DOMAINE DE L'INVENTION

L'invention concerne un réservoir de liquide biopharmaceutique, en particulier destiné au mélange de produits biopharmaceutiques ou plus particulièrement à la culture cellulaire comprenant une poche, un ensemble de pièces fixe par rapport à la poche, un ensemble de pièces tournant autour d'un axe de rotation.

### ARRIERE PLAN TECHNOLOGIQUE DE L'INVENTION

Des réservoirs d'agitation ou de mélange de liquide biopharmaceutique utilisent un agitateur par exemple rotatif pour mélanger entre eux des composés chimiques. Souvent, les constituants à mélanger requièrent un environnement stérile, comme c'est le cas pour préparer un produit pharmaceutique. Pour garantir la stérilité de l'environnement, les réservoirs de mélange sont structurés de manière à empêcher les éléments contaminants extérieurs de rentrer dans le réservoir, et ceci tout au long du processus, que ce soit pendant le remplissage, pendant le mélange ou encore pendant la vidange du liquide biopharmaceutique.

Selon un premier art antérieur, il existe un réservoir de liquide biopharmaceutique dont l'essentiel de l'organe mécanique, et en particulier le palier de l'arbre tournant, est situé à l'extérieur de la poche. Un inconvénient de ce premier art antérieur est de nécessiter la traversée de la paroi de la poche par l'arbre tournant muni d'un joint tournant ou d'un joint fixe à frottements. Ce joint tournant est complexe, coûteux et délicat. Ce joint fixe à frottements d'une part s'use vite, doit donc être remplacé souvent, et d'autre part crée beaucoup de particules lesquelles vont polluer le liquide biopharmaceutique. L'un ou l'autre de ces joints impose une vitesse de rotation limitée de l'arbre tournant.

Selon un deuxième art antérieur, il existe un réservoir de liquide biopharmaceutique dont l'essentiel de l'organe mécanique, et en particulier le palier de l'arbre tournant, est situé à l'intérieur de la poche. Un inconvénient de ce deuxième art antérieur est de n'être séparé de l'espace intérieur de stockage du liquide biopharmaceutique que par un couloir droit, par ailleurs vite entièrement rempli de liquide biopharmaceutique, favorisant ainsi d'une part la migration des particules d'usure du palier allant polluer l'espace intérieur de stockage du liquide biopharmaceutique et d'autre part la migration des cellules et de leurs supports ou micro-supports venant se faire broyer dans le palier risquant d'abîmer le pallier et de polluer l'espace intérieur de stockage du liquide biopharmaceutique avec les débris broyés des micro-supports migrant dans l'autre sens ensuite. Enfin la destruction d'une partie des cellules cultivées pénalise d'autant le rendement de la culture cellulaire.

US5385546 divulgue un réservoir de liquide biopharmaceutique, comprenant: une poche formant un espace intérieur de stockage du liquide biopharmaceutique, un organe mécanique situé au niveau d'une paroi de la poche, comprenant un ensemble de pièces, fixe par rapport à la paroi de la poche, un ensemble de pièces, tournant autour d'un axe de rotation par rapport à l'ensemble fixe, un palier à l'intérieur de la poche, et un passage de communication séparant le palier de l'espace intérieur de stockage, comprenant un ou plusieurs changements de direction.

### OBJETS DE L'INVENTION

Le but de la présente invention est de fournir un réservoir de liquide biopharmaceutique palliant au moins partiellement les inconvénients précités.

Plus particulièrement, l'invention vise à fournir un réservoir de liquide biopharmaceutique qui, d'une part localise l'essentiel de l'organe mécanique, et en particulier, le palier et l'organe ou élément tournant supporté par ce palier, à l'intérieur de la poche, évitant ainsi un joint délicat et une vitesse de rotation trop limitée de l'organe ou élément tournant, et d'autre part évite ou au moins réduit notablement la communication entre d'une part l'entourage du palier, source de particules d'usure, et d'autre part l'espace intérieur de stockage de liquide biopharmaceutique.

Cette suppression, ou au moins cette réduction de communication, entre l'entourage du palier et l'espace intérieur de stockage de liquide biopharmaceutique, évite ou au moins réduit ainsi d'une part la contamination du liquide biopharmaceutique stocké par les particules d'usure du palier, et d'autre part le broyage du contenu du liquide biopharmaceutique, cellules et éléments de support, détériorant le palier et risquant de polluer le liquide biopharmaceutique stocké lors de leur retour dans l'espace intérieur de stockage de ce liquide biopharmaceutique dans la poche du réservoir.

A cette fin, la présente invention propose un réservoir de liquide biopharmaceutique, comprenant : une poche formant un espace intérieur de stockage du liquide biopharmaceutique, un organe mécanique situé au niveau d'une paroi de la poche, comprenant : un ensemble de pièces, fixe par rapport à la paroi de la poche, un ensemble de pièces, tournant autour d'un axe de rotation par rapport à l'ensemble fixe, un palier situé entre les deux ensembles et à l'intérieur de la poche, un passage de communication : séparant le palier de l'espace intérieur de stockage, comprenant un ou plusieurs changements de direction, étant formé par une partie des pièces de l'ensemble tournant située en regard d'une partie des pièces de l'ensemble fixe. En fait cela signifie que le passage de communication est formé, d'une part par une partie des pièces de l'ensemble tournant, et d'autre part par une partie des pièces de l'ensemble fixe, ladite partie des pièces de l'ensemble tournant étant située en regard de ladite partie des pièces de l'ensemble fixe.

A cette fin, l'invention propose également un organe mécanique pour réservoir de liquide biopharmaceutique lequel comprend une poche de stockage du liquide biopharmaceutique, caractérisé en ce qu'il comprend : un ensemble de pièces, fixe, un ensemble de pièces, tournant autour d'un axe de rotation par rapport à l'ensemble fixe, un palier situé entre les deux ensembles, un passage de communication : séparant le palier de l'extérieur de l'organe mécanique, comprenant un ou plusieurs changements de direction, étant formé par une partie des pièces de l'ensemble tournant située en regard d'une partie des pièces de l'ensemble fixe.

De préférence, le passage forme un siphon inversé, de l'air étant piégé dans le fond du siphon inversé par du liquide situé au moins dans une branche du siphon inversé partant du fond du siphon inversé et débouchant dans l'espace intérieur de stockage.

Ainsi, la présence du siphon dans le passage de communication permet de réduire encore bien plus la communication, entre l'entourage du palier et l'espace intérieur de stockage de liquide biopharmaceutique. Le siphon est inversé car c'est de l'air piégé au fond du siphon par du liquide dans l'une et/ou l'autre des branches du siphon s'étendant de part et d'autre du fond du siphon, au lieu d'être du liquide piégé par de l'air comme dans un siphon d'évacuation sanitaire classique.

De préférence, ledit air étant piégé dans le fond du siphon inversé par du liquide situé au moins dans les deux branches du siphon inversé, la branche partant du fond du siphon inversé et débouchant dans l'espace intérieur de stockage et la branche partant du fond du siphon inversé et allant jusqu'au palier.

Ainsi, l'air étant piégé de chaque côté par un bouchon de liquide, la communication entre l'entourage du palier et l'espace intérieur de stockage de liquide biopharmaceutique est encore plus réduite, voire même peut être pratiquement supprimée.

De préférence, le passage de communication chemine majoritairement entre deux pièces qui sont d'une part un chapeau de l'ensemble tournant et une cuvette de l'ensemble fixe, le chapeau tournant autour de l'axe de rotation par rapport à la cuvette, le chapeau et la cuvette étant imbriquées au moins partiellement l'un dans l'autre pour former le passage de communication.

Ainsi, l'espace situé entre les ensembles tournant et fixe, point faible du réservoir, est aménagé de manière à supprimer ou au moins à réduire la possibilité de communication entre l'entourage du palier et l'espace intérieur de stockage de liquide biopharmaceutique, les autres espaces pouvant plus facilement être rendus étanches, préférentiellement par simple clipsage d'une pièce sur une autre ou d'une pièce dans une autre.

De préférence, l'un des ensembles forme une protubérance rentrant dans un creux de l'autre ensemble, le passage de communication contournant la protubérance en cheminant le long des parois du creux.

Ainsi, la communication entre l'entourage du palier et l'espace intérieur de stockage de liquide biopharmaceutique est encore réduite, et ceci de manière simple. En effet, une simple pénétration d'une pièce mâle dans une pièce femelle permet de réaliser une chicane réduisant de manière efficace la communication entre l'entourage du palier et l'espace intérieur de stockage de liquide biopharmaceutique.

De préférence, l'ensemble tournant forme le creux et l'ensemble fixe forme la protubérance.

Ainsi, l'air est piégé plus efficacement, dans le fond du siphon situé plus haut que les branches du siphon, l'organe mécanique étant alors disposé en partie basse du réservoir, avantageusement au niveau de la paroi inférieure de la poche.

De préférence, le passage de communication est un passage présentant une symétrie de révolution autour de l'axe de rotation.

Ainsi, l'encombrement autour de l'axe de rotation est minimisé.

De préférence, la largeur du passage de communication dans un plan contenant l'axe de rotation est comprise entre 0.5mm et 5mm, de préférence compris entre 1mm et 3mm.

Ainsi, la rotation de l'ensemble tournant autour de l'ensemble fixe est mieux sécurisée, l'espace étant suffisamment large pour garantir l'absence de frottement ou d'accrochage lors de la rotation, tout en réduisant notablement la communication entre l'entourage du palier et l'espace intérieur de stockage de liquide biopharmaceutique, l'espace étant également suffisant étroit pour bien piéger l'air dans le fond du siphon et bien gêner voire empêcher la migration d'éléments indésirables de l'un des côtés vers l'autre côté, particules d'usure du palier vers l'espace intérieur de stockage, ou cellules et supports vers le voisinage du palier.

De préférence, le passage de communication comprend au moins deux couloirs parallèles à l'axe de rotation, séparés par un virage.

Ainsi, la présence de cette chicane réduit bien la communication entre l'entourage du palier et l'espace intérieur de stockage de liquide biopharmaceutique.

De préférence, l'un des couloirs parallèles à l'axe de rotation relie le virage à une cavité comprenant le palier.

Ainsi, cette chicane très simple et très courte, déjà suffisante, réduit encore le risque de frottement ou d'accrochage pendant la rotation de l'ensemble tournant autour de l'ensemble fixe, même après un temps important lorsque ces deux ensembles présentent déjà des marques d'usure dues à la rotation.

De préférence, le passage de communication comprend des ailettes radiales, avantageusement régulièrement réparties autour de l'axe de rotation, avantageusement situées dans la branche du siphon inversé partant du fond du siphon inversé et débouchant dans l'espace intérieur de stockage, avantageusement au nombre de 8 à 15 ailettes. Avantageusement, ces ailettes radiales ne s'étendent que sur une partie de la largeur du passage de communication.

Ainsi, la présence de ces ailettes radiales permet de mieux piéger d'éventuelles particules solides qui se trouveraient situées dans le passage de communication formant siphon inversé, ou d'empêcher ou de gêner de telles particules solides qui auraient tendance à entrer dans le passage de communication formant siphon inversé ou encore à le traverser.

De préférence, les ailettes radiales s'étendent, avantageusement perpendiculairement, à partir de la face externe de la paroi interne d'une partie de l'ensemble fixe.

Ainsi, la présence de ces ailettes radiales perturbe moins l'hydrodynamique du liquide situé dans le passage de communication formant siphon inversé. Alternativement, les ailettes radiales peuvent s'étendre, avantageusement perpendiculairement, à partir de la face interne de la paroi externe d'une partie de l'ensemble tournant.

De préférence, les ailettes radiales s'étendent de manière inclinée par rapport à une paroi de l'intérieur du passage de communication, le sens de l'inclinaison étant tel que compte-tenu du sens de rotation de l'ensemble tournant autour de l'ensemble fixe d'éventuelles particules solides situées dans le passage de communication restent retenues ou bloquées entre les ailettes et ladite paroi de l'intérieur du passage de communication.

Alternativement, les ailettes radiales peuvent s'étendre de manière droite par rapport à une paroi de l'intérieur du passage de communication formant siphon inversé.

En variantes :
➢ ces ailettes peuvent être des plaques plates parallèles à l'axe de rotation,
➢ ces ailettes peuvent être des plaques incurvées et inclinées globalement par rapport à l'axe de rotation avec un angle aigu, préférentiellement inférieur à 30 degrés, avantageusement de l'ordre de 15 degrés,
➢ ces ailettes peuvent être des plaques droites inclinées par rapport à l'axe de rotation avec un angle aigu, préférentiellement inférieur à 30 degrés, avantageusement de l'ordre de 15 degrés.

De préférence, le palier est la seule zone de support de l'ensemble tournant par l'ensemble fixe.

Ainsi, la réduction de la communication entre l'entourage du palier et l'espace intérieur de stockage de liquide biopharmaceutique y est donc particulièrement critique.

De préférence, le palier est la seule zone de contact entre l'ensemble tournant et l'ensemble fixe.

Ainsi, la réduction de la communication entre l'entourage du palier et l'espace intérieur de stockage de liquide biopharmaceutique y est donc particulièrement critique.

De préférence, le palier est un roulement à billes.

Ainsi, la réduction de la communication entre l'entourage du palier et l'espace intérieur de stockage de liquide biopharmaceutique est particulièrement intéressante car le roulement à billes est particulièrement apte à broyer en petits morceaux tous les petits éléments, comme les cellules du liquide biopharmaceutiques ou leurs supports, ces petits morceaux étant particulièrement aptes à retourner polluer le liquide biopharmaceutique stocké et étant particulièrement difficiles à filtrer lors d'une vidange partielle ou totale subséquente de la poche, une fois que les cellules se sont suffisamment multipliées dans le liquide biopharmaceutique stocké dans l'espace intérieur de stockage de la poche.

De préférence, l'organe mécanique traverse la paroi de la poche au niveau d'une ouverture de la paroi de la poche.

Ainsi, le maintien du palier à l'intérieur de la poche est particulièrement avantageux, car il évite tout joint complexe et délicat, comme un joint tournant, présent dans le premier art antérieur.

De préférence, l'ouverture de la paroi de la poche est fixée de manière étanche autour de l'organe mécanique.

Ainsi, toute fuite directe du liquide biopharmaceutique stocké dans la poche vers l'extérieur de la poche tout comme toute pollution de ce liquide biopharmaceutique stocké par des particules en provenance directe de l'extérieur de la poche, peuvent être évitées.

De préférence, l'ouverture est soudée ou collée autour de l'ensemble fixe.

Ainsi, cette étanchéité entre la poche et l'extérieur de la poche, au niveau de l'organe mécanique, est réalisée simplement.

De préférence, l'ensemble tournant porte un arbre de rotation.

Ainsi, un arbre de rotation requérant une vitesse relativement élevée de rotation de l'ensemble tournant autour de l'ensemble fixe, il est particulièrement intéressant de réduire la communication entre l'entourage du palier et l'espace intérieur de stockage de liquide biopharmaceutique.

De préférence, l'arbre de rotation porte lui-même une hélice.

Ainsi, cette hélice entraînant des risques accrus de pollution en raison des vibrations importantes entraînées, il est particulièrement intéressant de réduire la communication entre l'entourage du palier et l'espace intérieur de stockage de liquide biopharmaceutique.

De préférence, l'ensemble fixe comprend un port de vidange débouchant à l'extérieur de la poche.

Ainsi, l'étanchéité de l'organe mécanique au niveau de la paroi de la poche est d'autant plus importante à maintenir.

De préférence, un distributeur de gaz est situé autour des ensembles, dans l'espace intérieur de stockage.

Ainsi, ce distributeur de gaz entraînant une augmentation de la circulation du liquide biopharmaceutique au voisinage de l'organe mécanique, il est d'autant plus important de réduire la communication entre l'entourage du palier et l'espace intérieur de stockage de liquide biopharmaceutique.

De préférence, le distributeur de gaz est annulaire.

Ainsi, l'augmentation de la circulation du liquide biopharmaceutique au voisinage de l'organe mécanique est même réalisée tout autour de l'organe mécanique, il est alors d'autant plus important de réduire la communication entre l'entourage du palier et l'espace intérieur de stockage de liquide biopharmaceutique.

De préférence, le distributeur de gaz comprend une dérivation apte à souffler de l'air dans le passage de communication de manière à tendre à le faire ressortir dans l'espace intérieur de stockage.

Ainsi, cette dérivation va aider à améliorer la réduction de la communication entre l'entourage du palier et l'espace intérieur de stockage de liquide biopharmaceutique, en refoulant le liquide biopharmaceutique dans l'espace intérieur de stockage d'où il vient.

De préférence, l'ensemble tournant comprend une pièce intermédiaire de liaison portant l'arbre de rotation et le palier.

Ainsi, la présence de cette pièce intermédiaire de liaison permet plus facilement d'aménager l'entourage du palier de manière à pouvoir ensuite plus facilement réduire la communication entre l'entourage du palier et l'espace intérieur de stockage de liquide biopharmaceutique.

De préférence, l'arbre de rotation est clipsé dans la pièce intermédiaire de liaison.

Ainsi, l'étanchéité à cet endroit est plus facilement réalisée de manière à la fois simple et efficace.

De préférence, le palier comprend une bague tournante, de préférence la bague extérieure du palier, qui clipsée dans la pièce intermédiaire de liaison.

Ainsi, l'étanchéité à cet endroit est plus facilement réalisée de manière à la fois simple et efficace.

De préférence, la pièce intermédiaire de liaison porte le chapeau tournant qui est clipsé sur cette pièce intermédiaire de liaison.

Ainsi, l'étanchéité à cet endroit est plus facilement réalisée de manière à la fois simple et efficace.

De préférence, l'ensemble fixe comprend un socle qui porte le palier et qui présente un port de vidange débouchant à l'extérieur de l'espace intérieur de stockage.

Ainsi, il est particulièrement intéressant de bien garantir l'étanchéité entre intérieur et extérieur de la poche au niveau de l'organe mécanique.

De préférence, le distributeur de gaz est clipsé autour du socle.

Ainsi, l'étanchéité à cet endroit est plus facilement réalisée de manière à la fois simple et efficace.

De préférence, le palier est clipsé autour du socle.

Ainsi, l'étanchéité à cet endroit est plus facilement réalisée de manière à la fois simple et efficace.

De préférence, le palier comprend une bague fixe, qui est de préférence la bague intérieure du palier, qui maintient la cuvette bloquée contre le socle.

Ainsi, l'étanchéité à cet endroit est plus facilement réalisée de manière à la fois simple et efficace.

De préférence, le liquide biopharmaceutique comprend : des micro-supports inertes, dont la plus grande dimension est inférieure à 0.3mm, de préférence inférieure à 0.1mm, avantageusement supérieure à 10µm, des cellules accrochées à ces micro-supports.

Ainsi, il est particulièrement intéressant de réduire la communication entre l'entourage du palier et l'espace intérieur de stockage de liquide biopharmaceutique, puisque la très faible taille des éléments solides du liquide biopharmaceutique stocké les rend particulièrement aptes à se déplacer partout, et en particulier à venir glisser jusque dans le palier s'ils le pouvaient.

De préférence, les micro-supports sont des billes.

Ainsi, ces éléments sphériques du liquide biopharmaceutique stocké se déplacent encore plus facilement, et même dans les endroits exigus ; d'où l'intérêt de réduire encore la communication entre l'entourage du palier et l'espace intérieur de stockage de liquide biopharmaceutique.

De préférence, la majorité des pièces des ensembles sont en plastique rigide, légèrement déformables de manière à pouvoir réaliser une opération de clipsage avec elles, sont de préférence en polyéthylène (PE).

Ainsi, l'étanchéité à cet endroit est plus facilement réalisée de manière à la fois simple et efficace, tout en permettant aisément à l'organe mécanique de remplir sa fonction mécanique, ici en particulier une fonction mécanique de rotation requérant une certaine rigidité de ses pièces constitutives.

De préférence, la paroi de la poche est en matériau suffisamment souple et déformable pour pouvoir être pliée.

Ainsi, l'étanchéité au niveau d'une paroi, et en particulier d'une ouverture dans la paroi, est d'autant plus intéressante à réaliser.

Un palier est un organe mécanique supportant et guidant un organe mécanique tournant, de préférence un arbre tournant.

Un clips est un système de fixation intégré ou non à la pièce, qui se déforme élastiquement lors de son insertion, et qui après insertion, ne subit généralement plus aucune contrainte.

Un clipsage est une opération d'assemblage dans laquelle la déformation élastique d'une ou des deux pièces impliquées durant la phase d'insertion permet leur attachement après retour élastique. Le clipsage réalise un emboîtement de pièces utilisant la déformation élastique de certains éléments, par exemple d'une pièce femelle, souvent en matière plastique, et comprend avantageusement une ou plusieurs griffes (ou un ou plusieurs ergots) dont la forme peut permettre un démontage facile et rapide.

D'autres caractéristiques et avantages de l'invention apparaîtront à la lecture de la description qui suit d'un mode de réalisation préféré de l'invention, donnée à titre d'exemple et en référence aux dessins annexés.

### BREVE DESCRIPTION DES DESSINS

**[****Fig 1****]** La figure 1 représente schématiquement une vue globale en perspective coupée d'un exemple d'assemblage des ensembles fixe et tournant de l'organe mécanique du réservoir de liquide biopharmaceutique selon un premier mode de réalisation de l'invention.
**[****Fig 2****]** La figure 2 représente schématiquement une vue globale en perspective coupée, d'un détail optionnel montrant des ailettes dans le passage de communication, d'un exemple d'assemblage des ensembles fixe et tournant de l'organe mécanique du réservoir de liquide biopharmaceutique selon un premier mode de réalisation de l'invention.
**[****Fig 3****]** La figure 3 représente schématiquement une vue globale en perspective coupée, d'un détail optionnel montrant des ailettes dans le passage de communication, d'un exemple d'assemblage des ensembles fixe et tournant de l'organe mécanique du réservoir de liquide biopharmaceutique selon un premier mode de réalisation de l'invention.
**[****Fig 4****]** La figure 4 représente schématiquement une vue globale en perspective coupée, d'un détail optionnel montrant des ailettes dans le passage de communication, d'un exemple d'assemblage des ensembles fixe et tournant de l'organe mécanique du réservoir de liquide biopharmaceutique selon un premier mode de réalisation de l'invention.
**[****Fig 5****]** La figure 5 représente schématiquement une vue globale en perspective coupée, d'un détail optionnel montrant des ailettes dans le passage de communication, d'un exemple d'assemblage des ensembles fixe et tournant de l'organe mécanique du réservoir de liquide biopharmaceutique selon un premier mode de réalisation de l'invention.
**[****Fig 6****]** La figure 6 représente schématiquement une vue globale en perspective coupée, d'un détail optionnel montrant des ailettes dans le passage de communication, d'un exemple d'assemblage des ensembles fixe et tournant de l'organe mécanique du réservoir de liquide biopharmaceutique selon un premier mode de réalisation de l'invention.
**[****Fig 7****]** La figure 7 représente schématiquement une vue globale éclatée en perspective coupée d'un exemple d'assemblage des ensembles fixe et tournant de l'organe mécanique du réservoir de liquide biopharmaceutique selon un premier mode de réalisation de l'invention.
**[****Fig 8****]** La figure 8 représente schématiquement une vue globale en perspective d'un exemple d'assemblage des ensembles fixe et tournant de l'organe mécanique du réservoir de liquide biopharmaceutique selon un premier mode de réalisation de l'invention.
**[****Fig 9****]** La figure 9 représente schématiquement une vue globale en perspective coupée d'un exemple d'ensemble tournant de l'organe mécanique du réservoir de liquide biopharmaceutique selon un premier mode de réalisation de l'invention.
**[****Fig 10****]** La figure 10 représente schématiquement une vue locale en perspective d'un exemple d'une partie d'ensemble tournant de l'organe mécanique du réservoir de liquide biopharmaceutique selon un premier mode de réalisation de l'invention.
**[****Fig 11****]** La figure 11 représente schématiquement une vue globale en perspective coupée d'un exemple d'ensemble tournant de l'organe mécanique du réservoir de liquide biopharmaceutique selon un premier mode de réalisation de l'invention.
**[****Fig 12****]** La figure 12 représente schématiquement une vue locale en perspective d'un exemple d'une partie d'ensemble tournant de l'organe mécanique du réservoir de liquide biopharmaceutique selon un premier mode de réalisation de l'invention.
**[****Fig 13****]** La figure 13 représente schématiquement une vue locale en perspective d'un exemple d'une partie d'ensemble tournant de l'organe mécanique du réservoir de liquide biopharmaceutique selon un premier mode de réalisation de l'invention.
**[****Fig 14****]** La figure 14 représente schématiquement une vue globale en perspective coupée d'un exemple d'ensemble tournant de l'organe mécanique du réservoir de liquide biopharmaceutique selon un premier mode de réalisation de l'invention.
**[****Fig 15****]** La figure 15 représente schématiquement une vue locale en perspective d'un exemple d'une partie d'ensemble tournant de l'organe mécanique du réservoir de liquide biopharmaceutique selon un premier mode de réalisation de l'invention.
**[****Fig 16****]** La figure 16 représente schématiquement une vue locale en perspective d'un exemple d'une partie d'ensemble tournant de l'organe mécanique du réservoir de liquide biopharmaceutique selon un premier mode de réalisation de l'invention.
**[****Fig 17****]** La figure 17 représente schématiquement une vue globale en perspective coupée d'un exemple d'ensemble fixe de l'organe mécanique du réservoir de liquide biopharmaceutique selon un premier mode de réalisation de l'invention.
**[****Fig 18****]** La figure 18 représente schématiquement une vue globale en perspective coupée d'un exemple d'ensemble fixe de l'organe mécanique du réservoir de liquide biopharmaceutique selon un premier mode de réalisation de l'invention.
**[****Fig 19****]** La figure 19 représente schématiquement une vue locale en perspective coupée d'un exemple d'une partie d'ensemble fixe de l'organe mécanique du réservoir de liquide biopharmaceutique selon un premier mode de réalisation de l'invention.
**[****Fig 20****]** La figure 20 représente schématiquement une vue globale en perspective coupée d'un exemple d'ensemble fixe de l'organe mécanique du réservoir de liquide biopharmaceutique selon un premier mode de réalisation de l'invention.
**[****Fig 21****]** La figure 21 représente schématiquement une vue locale en perspective coupée d'un exemple d'une partie d'ensemble fixe de l'organe mécanique du réservoir de liquide biopharmaceutique selon un premier mode de réalisation de l'invention.
**[****Fig 22****]** La figure 22 représente schématiquement une vue globale en perspective coupée d'un exemple d'ensemble fixe de l'organe mécanique du réservoir de liquide biopharmaceutique selon un premier mode de réalisation de l'invention.
**[****Fig 23****]** La figure 23 représente schématiquement une vue globale en perspective coupée d'un exemple d'assemblage des ensembles fixe et tournant de l'organe mécanique du réservoir de liquide biopharmaceutique selon un premier mode de réalisation de l'invention, montrant la dérivation reliant le distributeur de gaz et le passage de communication.
**[****Fig 24****]** La figure 24 représente schématiquement une vue globale en perspective coupée d'un exemple d'assemblage des ensembles fixe et tournant de l'organe mécanique du réservoir de liquide biopharmaceutique selon un deuxième mode de réalisation de l'invention.
**[****Fig 25****]** La figure 25 représente schématiquement une vue globale éclatée en perspective coupée d'un exemple d'assemblage des ensembles fixe et tournant de l'organe mécanique du réservoir de liquide biopharmaceutique selon un deuxième mode de réalisation de l'invention.
**[****Fig 26****]** La figure 26 représente schématiquement une vue globale en perspective d'un exemple d'assemblage des ensembles fixe et tournant de l'organe mécanique du réservoir de liquide biopharmaceutique selon un deuxième mode de réalisation de l'invention.
**[****Fig 27****]** La figure 27 représente schématiquement une vue globale en perspective coupée d'un exemple d'ensemble fixe de l'organe mécanique du réservoir de liquide biopharmaceutique selon un deuxième mode de réalisation de l'invention.
**[****Fig 28****]** La figure 28 représente schématiquement une vue locale en perspective coupée d'un exemple d'une partie d'ensemble fixe de l'organe mécanique du réservoir de liquide biopharmaceutique selon un deuxième mode de réalisation de l'invention.
**[****Fig 29****]** La figure 29 représente schématiquement une vue globale en perspective coupée d'un exemple d'ensemble tournant de l'organe mécanique du réservoir de liquide biopharmaceutique selon un deuxième mode de réalisation de l'invention.
**[****Fig 30****]** La figure 30 représente schématiquement une vue locale en perspective coupée d'un exemple d'une partie d'ensemble tournant de l'organe mécanique du réservoir de liquide biopharmaceutique selon un deuxième mode de réalisation de l'invention.
**[****Fig 31****]** La figure 31 représente schématiquement une vue globale en perspective coupée d'un exemple d'ensemble tournant de l'organe mécanique du réservoir de liquide biopharmaceutique selon un deuxième mode de réalisation de l'invention.
**[****Fig 32****]** La figure 32 représente schématiquement une vue locale en perspective coupée d'un exemple d'une partie d'ensemble tournant de l'organe mécanique du réservoir de liquide biopharmaceutique selon un deuxième mode de réalisation de l'invention.
**[****Fig 33****]** La figure 33 représente schématiquement une vue globale en perspective coupée d'un exemple d'ensemble fixe de l'organe mécanique du réservoir de liquide biopharmaceutique selon un deuxième mode de réalisation de l'invention.

### DESCRIPTION DE MODES DE REALISATION PREFERES DE L'INVENTION

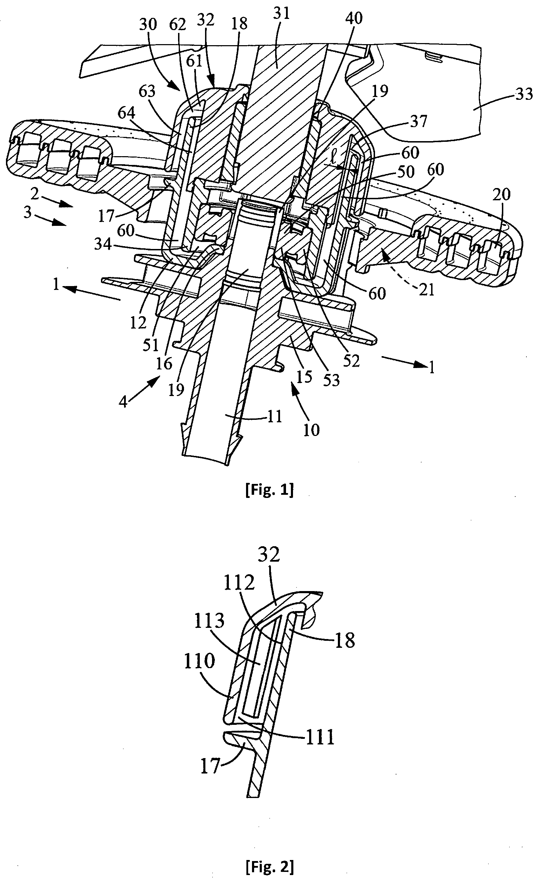

Les figures 1 à 22 représentent un premier mode de réalisation de l'invention, correspondant avantageusement à un volume de poche compris entre 50 et 200 litres.

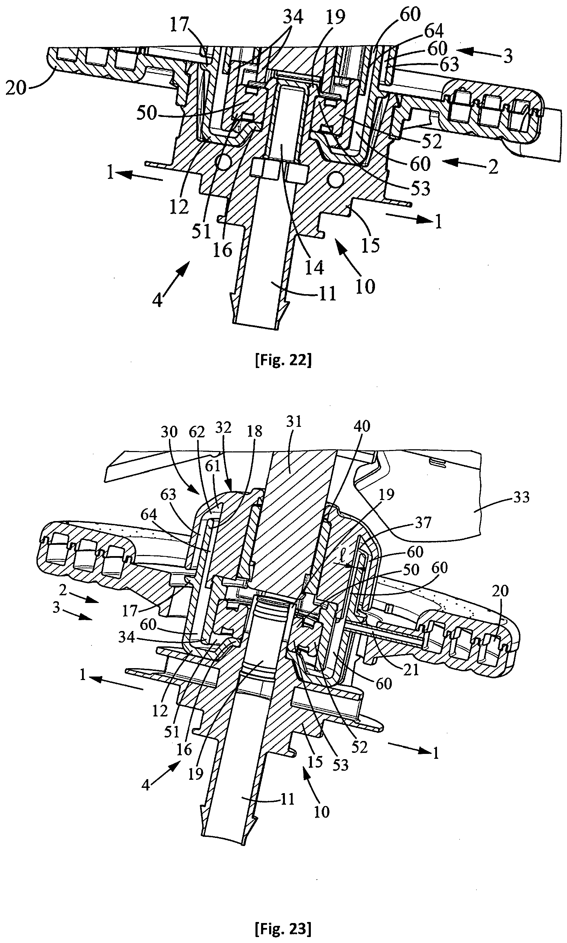

Les figures 23 à 33 représentent un deuxième mode de réalisation de l'invention, correspondant avantageusement à un volume de poche compris entre 500 et 1000 litres.

La figure 1 représente schématiquement une vue globale en perspective coupée d'un exemple d'assemblage des ensembles fixe et tournant de l'organe mécanique du réservoir de liquide biopharmaceutique selon un premier mode de réalisation de l'invention.

Le réservoir de liquide biopharmaceutique comprend une poche 1 et un organe mécanique 4. L'organe mécanique 4 est fixé au niveau d'une ouverture dans la paroi de la poche 1 laquelle n'est pas représentée sur la figure 1 mais s'étend autour de l'organe mécanique 4 dans les directions des flèches 1. La poche 1 forme un espace intérieur 2 de stockage d'un liquide biopharmaceutique 3. L'organe mécanique 4 traverse la paroi de la poche 1.

L'organe mécanique 4 comprend un ensemble 10 de pièces, fixe par rapport à la paroi de la poche 1, et un ensemble 30 de pièces, tournant autour d'un axe de rotation 31 par rapport à l'ensemble fixe 10. Un palier 50 est situé d'une part entre les deux ensembles 10 et 30 et d'autre part à l'intérieur 2 de la poche 1.

Un passage 60 de communication sépare le palier 50 de l'espace intérieur 2 de stockage. Ce passage 60 de communication comprend un ou plusieurs changements de direction, par exemple ici de l'extérieur vers l'intérieur, ce passage 60 de communication commence au niveau de la petite protubérance 17 par un petit couloir horizontal vers l'intérieur, puis par un assez grand couloir montant, puis par un petit couloir horizontal vers l'intérieur (en fait c'est un virage), puis par un assez grand couloir descendant, et enfin par un petit couloir horizontal vers l'intérieur, avant d'arriver dans la cavité 51 où se trouve le palier 50. Ce passage 60 de communication est formé par une partie des pièces de l'ensemble tournant 30 située en regard d'une partie des pièces de l'ensemble fixe 10.

Ce passage 60 forme un siphon inversé 61, de l'air étant piégé dans le fond 62 du siphon inversé 61 par du liquide situé au moins dans une branche 63 du siphon inversé 61 partant du fond 62 du siphon inversé 61 et débouchant dans l'espace intérieur 2 de stockage. L'air est piégé dans le fond 62 du siphon inversé 61 par du liquide de préférence situé au moins dans les deux branches 63 et 64 du siphon inversé 61, la branche 63 partant du fond 62 du siphon inversé 61 et débouchant dans l'espace intérieur 2 de stockage et la branche 64 partant du fond 62 du siphon inversé 61 et allant jusqu'au palier 50. Ce siphon inversé 61, dont le fond 62 contient de l'air et dont les branches 63 et 64 contiennent du liquide, éventuellement du liquide biopharmaceutique 3, permet de bloquer le passage de ce liquide biopharmaceutique, d'une part de la branche 63 vers la branche 64, et d'autre part de la branche 64 vers la branche 63.

Ce passage de communication 60 chemine majoritairement entre deux pièces qui sont d'une part un chapeau 32 de l'ensemble tournant 30 et une cuvette 12 de l'ensemble fixe 10, le chapeau 32 tournant autour de l'axe de rotation 31 par rapport à la cuvette 12, le chapeau 32 et la cuvette 12 étant imbriquées au moins partiellement l'un dans l'autre pour former le passage de communication 60. L'un des ensembles 10 forme une protubérance 18 rentrant dans un creux 37 de l'autre ensemble 30, le passage de communication 60 contournant la protubérance 18 en cheminant le long des parois du creux 37. De préférence, l'ensemble tournant 30 forme le creux 37 et l'ensemble fixe 10 forme la protubérance 18.

Le passage de communication 60 est un passage présentant une symétrie de révolution autour de l'axe de rotation 31. La largeur l du passage de communication 60 dans un plan (par exemple le plan de la figure 1) contenant l'axe de rotation 31 est compris entre 0.5mm et 5mm, de préférence compris entre 1mm et 3mm. Le passage de communication 60 comprend au moins deux couloirs parallèles 63 et 64 à l'axe de rotation 31, séparés par un virage 62. L'un des 64 couloirs parallèles à l'axe de rotation 31 relie le virage 62 à une cavité 51 comprenant le palier 50. Le palier 50 est la seule zone de support de l'ensemble tournant 30 par l'ensemble fixe 10. Le palier 50 est même la seule zone de contact entre l'ensemble tournant 30 et l'ensemble fixe 10. Avantageusement, le palier est un roulement à billes.

L'organe mécanique 4 traverse la paroi de la poche 1 au niveau d'une ouverture de la paroi de la poche 1, laquelle poche 1 s'étend donc autour de l'organe mécanique 4 dans les directions des flèches 1. L'ouverture de la paroi de la poche 1 (non représentée sur la figure 1, mais dont l'emplacement est marqué par l'extension des flèches 1) est fixée de manière étanche autour de l'organe mécanique 4. Cette ouverture est soudée ou collée autour de l'ensemble fixe 10. L'ensemble tournant 30 porte un arbre de rotation 31. L'arbre de rotation 31 porte lui-même une hélice 33. L'hélice 33 peut par exemple tourner entre 10 et 500 tours par minute, par exemple à 50 tours par minute pour une poche 1 de 2000 litres de liquide biopharmaceutique 3 et par exemple à 250 tours par minute pour une poche 1 de 50 litres de liquide biopharmaceutique 3. Le liquide biopharmaceutique 3 est avantageusement un milieu cellulaire cultivé, encore appelé « culture cellulaire », qui va requérir une agitation assez forte pour se développer, entraînant une vitesse de rotation relativement élevée de l'arbre de rotation 31, ce qui rend le système d'isolation par siphon inversé 61 entre la cavité 51 du palier 50 d'une part et l'espace intérieur 2 de stockage du liquide biopharmaceutique 3 d'autre part, proposé par l'invention d'autant plus intéressant.

Un distributeur 20 de gaz est situé autour des ensembles 10 et 30, dans l'espace intérieur 2 de stockage. Ce distributeur 20 de gaz est annulaire. Ce distributeur 20 de gaz comprend une dérivation 21 apte à souffler de l'air dans le passage de communication 60 de manière à tendre à le faire ressortir dans l'espace intérieur 2 de stockage, ce qui contribue à refouler encore un peu plus le liquide biopharmaceutique 3 essayant de traverser de l'espace intérieur 2 de stockage vers la branche 63 du siphon inversé 61.

L'ensemble tournant 30 comprend une pièce intermédiaire de liaison 34 portant l'arbre de rotation 31 et le palier 50. L'arbre de rotation 31 est clipsé dans la pièce intermédiaire de liaison 34. Le palier 50 comprend une bague tournante 52, de préférence la bague extérieure 52 du palier 50, qui est clipsée dans la pièce intermédiaire de liaison 34. La pièce intermédiaire de liaison 34 porte le chapeau tournant 32 qui est clipsé sur cette pièce intermédiaire de liaison 34.

L'ensemble fixe 10 comprend un socle 15 qui porte le palier 50 et qui présente un port de vidange 11 débouchant à l'extérieur de l'espace intérieur 2 de stockage. Ce port de vidange 11 sert à la vidange partielle de la poche 1. Ce port de vidange 11 débouche à l'extérieur de la poche 1. C'est par ce port de vidange 11 qu'est introduite la pièce 14 de fixation de l'ensemble tournant 30 par rapport à l'ensemble fixe 10. Cette pièce 14 de fixation s'emmanche dans l'ensemble fixe 10 en écartant les parois d'une cheminée centrale 19 laquelle elle-même pousse sur le palier 50, fixant ainsi relativement les ensembles tournant 30 et fixe 10 l'un par rapport à l'autre. C'est cette pièce 14 de fixation qui maintient l'assemblage de l'ensemble tournant 30 sur l'ensemble fixe 10. Le distributeur 20 de gaz est clipsé autour du socle 15. Le palier 50 est clipsé autour du socle 15. Le palier 50 comprend une bague fixe 53, qui est de préférence la bague intérieure 53 du palier 50, qui maintient la cuvette 12 bloquée contre le socle 15 par l'intermédiaire de la lèvre intérieure 16 de la cuvette 12.

Le liquide biopharmaceutique 3 comprend des micro-supports inertes (non représentés sur la figure 1, mais ils sont uniformément répartis au sein du liquide biopharmaceutique 3), dont la plus grande dimension est inférieure à 0.3mm, de préférence inférieure à 0.1mm, avantageusement supérieure à 10µm, par exemple comprise entre 30µm et 80µm. Des cellules vivantes sont accrochées à ces micro-supports. Ces micro-supports sont avantageusement des billes. La contenance de la poche 1 peut aller de quelques dizaines de litres à quelques milliers de litres de liquide biopharmaceutique 3, préférentiellement de 50 litres à 2000 litres de liquide biopharmaceutique 3. Par exemple, toutes les 3 semaines ou tous les mois, la poche 1 subit une vidange partielle et un prélèvement du milieu cellulaire cultivé dans la poche 1. La paroi de la poche 1 est en matériau suffisamment souple et déformable pour pouvoir être pliée, par exemple la paroi de la poche 1 est en plastique souple.

La majorité des pièces des ensembles sont en plastique rigide, légèrement déformables de manière à pouvoir réaliser une opération de clipsage avec elles, sont de préférence en polyéthylène (PE), avantageusement en polyéthylène haute densité (HDPE), ou bien en polyéthylène de téréphtalate (PET). Sauf le palier 50, qui peut être réalisé en métal, toutes les autres pièces de l'organe mécanique 4 peuvent avantageusement être réalisées par moulage par injection. Préférentiellement, toutes les pièces des ensembles tournant 30 et fixe 10 peuvent être réalisées sans filetage, sans colle, et être assemblées les unes aux autres simplement par clipsage. Une lèvre 40 d'étanchéité directement surmoulée sur le chapeau 32, permet d'assurer l'étanchéité avec l'arbre de rotation 31 notamment lorsque celui-ci tourne car le chapeau 32 est solidaire de l'arbre de rotation 31 et tourne avec lui.

La figure 2 représente schématiquement une vue globale en perspective coupée d'un détail optionnel d'un exemple d'assemblage des ensembles fixe et tournant de l'organe mécanique du réservoir de liquide biopharmaceutique selon un premier mode de réalisation de l'invention.

La figure 2 montre des ailettes 113 régulièrement réparties autour de l'axe de rotation 31 dans la branche 63 du passage de communication 60 formant le siphon inversé 61, ces ailettes 113 s'étendant perpendiculairement à la face interne 111 de la paroi externe 110 du chapeau 32 de l'ensemble tournant 30, ces ailettes 113 étant des plaques plates parallèles à l'axe de rotation 31.

Il y a environ entre 8 et 15 ailettes régulièrement réparties autour de l'axe de rotation 31. Ces ailettes 113 s'étendent sur la moitié de la largeur l de la branche 63 du passage de communication 60. Ces ailettes 113 sont avantageusement venues de matière avec la face interne 111 de la paroi externe 110 du chapeau 32, mais alternativement elles peuvent être des plaques séparées soudées sur la face interne 111 de la paroi externe 110 du chapeau 32. Les ailettes 113 ne dépassent pas vers le bas de la paroi externe 110 du chapeau 32, elles s'arrêtent avantageusement avant la fin de cette paroi.

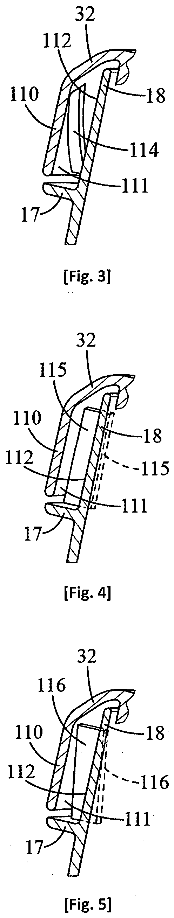

La figure 3 représente schématiquement une vue globale en perspective coupée d'un détail optionnel d'un exemple d'assemblage des ensembles fixe et tournant de l'organe mécanique du réservoir de liquide biopharmaceutique selon un premier mode de réalisation de l'invention.

La figure 3 montre des ailettes 114 régulièrement réparties autour de l'axe de rotation 31 dans la branche 63 du passage de communication 60 formant le siphon inversé 61, ces ailettes 114 s'étendant perpendiculairement à la face interne 111 de la paroi externe 110 du chapeau 32 de l'ensemble tournant 30, ces ailettes 114 étant des plaques incurvées et inclinées globalement par rapport à l'axe de rotation 31 avec un angle aigu, préférentiellement inférieur à 30 degrés, avantageusement de l'ordre de 15 degrés. Ces ailettes 114 pourraient également être des plaques droites inclinées par rapport à l'axe de rotation 31 avec un angle aigu, préférentiellement inférieur à 30 degrés, avantageusement de l'ordre de 15 degrés.

Il y a environ entre 8 et 15 ailettes 114 régulièrement réparties autour de l'axe de rotation 31. Ces ailettes 114 s'étendent sur la moitié de la largeur l de la branche 63 du passage de communication 60. Ces ailettes 114 sont avantageusement venues de matière avec la face interne 111 de la paroi externe 110 du chapeau 32, mais alternativement elles peuvent être des plaques séparées soudées sur la face interne 111 de la paroi externe 110 du chapeau 32. Les ailettes 114 ne dépassent pas vers le bas de la paroi externe 110 du chapeau 32, elles s'arrêtent avantageusement avant la fin de cette paroi.

La figure 4 représente schématiquement une vue globale en perspective coupée d'un détail optionnel d'un exemple d'assemblage des ensembles fixe et tournant de l'organe mécanique du réservoir de liquide biopharmaceutique selon un premier mode de réalisation de l'invention.

La figure 4 montre des ailettes 115 régulièrement réparties autour de l'axe de rotation 31 dans la branche 63 du passage de communication 60 formant le siphon inversé 61, ces ailettes 115 s'étendant perpendiculairement à la face externe 112 de la protubérance 18 de l'ensemble fixe 10, ces ailettes 115 étant des plaques droites parallèles à l'axe de rotation 31.

Il y a environ entre 8 et 15 ailettes 115 régulièrement réparties autour de l'axe de rotation 31. Ces ailettes 115 s'étendent sur la moitié de la largeur l de la branche 63 du passage de communication 60. Ces ailettes 115 sont avantageusement venues de matière avec la face externe 112 de la protubérance 18, mais alternativement elles peuvent être des plaques séparées soudées sur la face externe 112 de la protubérance 18. Les ailettes 115 reposent sur la petite protubérance 17.

La figure 5 représente schématiquement une vue globale en perspective coupée d'un détail optionnel d'un exemple d'assemblage des ensembles fixe et tournant de l'organe mécanique du réservoir de liquide biopharmaceutique selon un premier mode de réalisation de l'invention.

La figure 5 montre des ailettes 116 régulièrement réparties autour de l'axe de rotation 31 dans la branche 63 du passage de communication 60 formant le siphon inversé 61, ces ailettes 116 s'étendant perpendiculairement à la face externe 112 de la protubérance 18 de l'ensemble fixe 10, ces ailettes 116 étant des plaques droites inclinées par rapport à l'axe de rotation 31 avec un angle aigu, préférentiellement inférieur à 30 degrés, avantageusement de l'ordre de 15 degrés. Ces ailettes 116 pourraient également être des plaques incurvées et inclinées globalement par rapport à l'axe de rotation 31 avec un angle aigu, préférentiellement inférieur à 30 degrés, avantageusement de l'ordre de 15 degrés.

Il y a environ entre 8 et 15 ailettes 116 régulièrement réparties autour de l'axe de rotation 31. Ces ailettes 116 s'étendent sur la moitié de la largeur l de la branche 63 du passage de communication 60. Ces ailettes 116 sont avantageusement venues de matière avec la face externe 112 de la protubérance 18, mais alternativement elles peuvent être des plaques séparées soudées sur la face externe 112 de la protubérance 18. Les ailettes 116 reposent sur la petite protubérance 17.

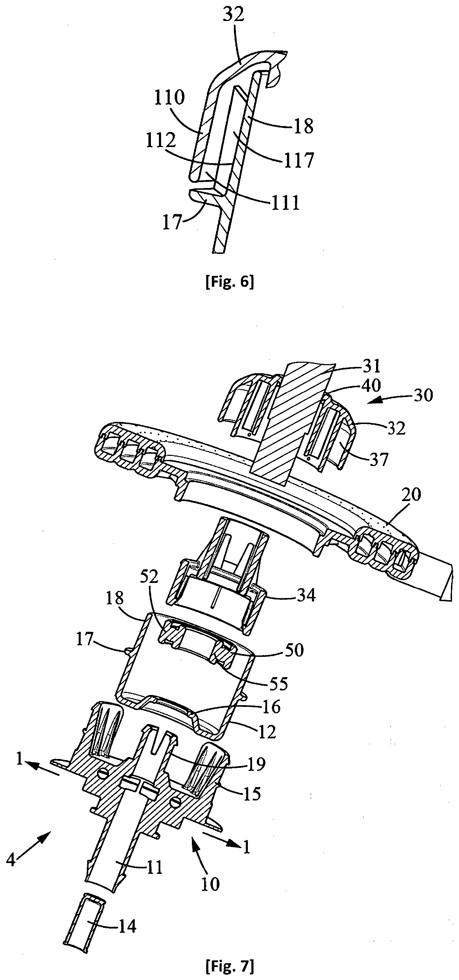

La figure 6 représente schématiquement une vue globale en perspective coupée d'un détail optionnel d'un exemple d'assemblage des ensembles fixe et tournant de l'organe mécanique du réservoir de liquide biopharmaceutique selon un premier mode de réalisation de l'invention.

La figure 6 montre des ailettes 117 régulièrement réparties autour de l'axe de rotation 31 dans la branche 63 du passage de communication 60 formant le siphon inversé 61, ces ailettes 117 s'étendant de manière inclinée par rapport à la face externe 112 de la protubérance 18 de l'ensemble fixe 10, le sens de l'inclinaison étant tel que compte-tenu du sens de rotation de l'ensemble tournant 30 autour de l'ensemble fixe 10 d'éventuelles particules solides situées dans la branche 63 du passage de communication 60 restent retenues ou bloquées entre les ailettes 117 et la paroi externe 112 de la protubérance 18, ces ailettes 117 étant des plaques droites parallèles à l'axe de rotation 31.

Il y a environ entre 8 et 15 ailettes 117 régulièrement réparties autour de l'axe de rotation 31. Ces ailettes 117 s'étendent sur la moitié de la largeur l de la branche 63 du passage de communication 60. Ces ailettes 117 sont avantageusement venues de matière avec la face externe 112 de la protubérance 18, mais alternativement elles peuvent être des plaques séparées soudées sur la face externe 112 de la protubérance 18. Les ailettes 117 reposent sur la petite protubérance 17.

La figure 7 représente schématiquement une vue globale éclatée en perspective coupée d'un exemple d'assemblage des ensembles fixe et tournant de l'organe mécanique du réservoir de liquide biopharmaceutique selon un premier mode de réalisation de l'invention.

Le chapeau 32 rentre dans la pièce intermédiaire 34. Le distributeur 20 de gaz est directement clipsé sur le socle 15. La pièce intermédiaire 34 rentre dans la cuvette 12 qui rentre dans le socle 15. La pièce de liaison 14 rentre dans le port de vidange 11, traverse le socle 15 jusqu'à arriver en haut de la cheminée 19, fixant ainsi l'ensemble tournant 30 par rapport à l'ensemble fixe 10, le palier 50 étant bloqué d'une part en haut par les ergots radiaux externes situés au sommet de la cheminée 19 et d'autre part en bas par la lèvre intérieure 16 de la cuvette 12.

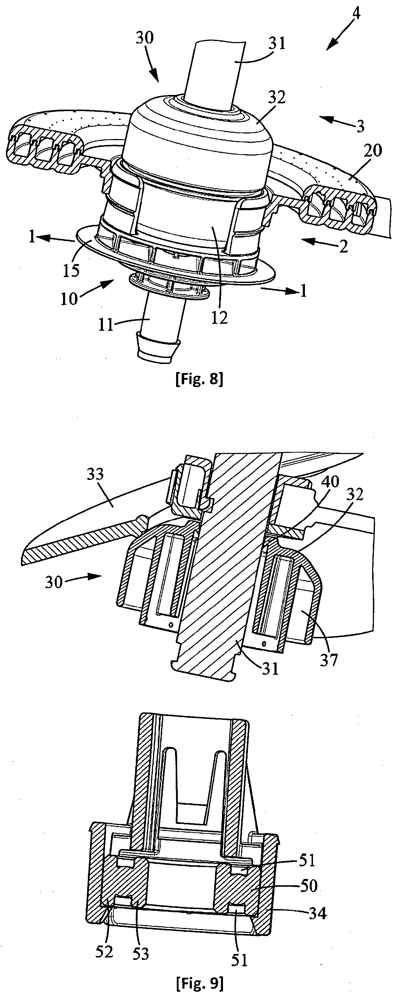

La figure 8 représente schématiquement une vue globale en perspective d'un exemple d'assemblage des ensembles fixe et tournant de l'organe mécanique du réservoir de liquide biopharmaceutique selon un premier mode de réalisation de l'invention. L'organe mécanique 4 est en position complètement montée.

La figure 9 représente schématiquement une vue globale en perspective coupée d'un exemple d'ensemble tournant de l'organe mécanique du réservoir de liquide biopharmaceutique selon un premier mode de réalisation de l'invention.

Dans une première étape de montage de l'organe mécanique 4, l'arbre de rotation 31 est d'abord emmanché dans le chapeau 32, grâce à une certaine souplesse de la lèvre d'étanchéité 40 (qui peut être en LLDPE par exemple).

Dans une deuxième étape de montage de l'organe mécanique 4, le palier 50 est clipsé dans le bas de la pièce intermédiaire 34, avantageusement à l'aide d'un outil ou bien par simple pression manuelle. La pièce intermédiaire présente à la fois une bonne rigidité et un peu de souplesse au niveau de ses clips en partie basse sous la bague externe 52 du palier 50.

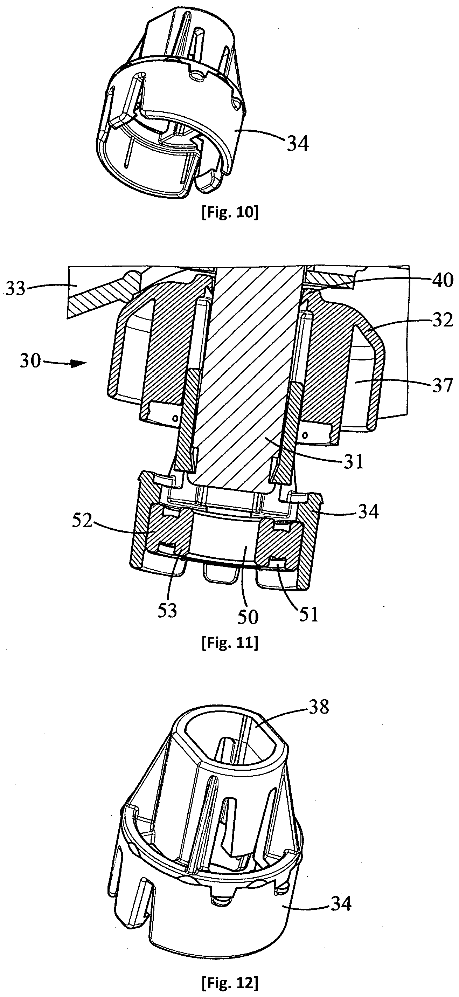

La figure 10 représente schématiquement une vue locale en perspective d'un exemple d'une partie d'ensemble tournant de l'organe mécanique du réservoir de liquide biopharmaceutique selon un premier mode de réalisation de l'invention.

La couronne intérieure basse de la pièce intermédiaire de liaison 34 comprend sur sa surface interne six protubérances disposées pour absorber les tolérances de fabrication et éviter une rotation libre entre la bague extérieure 52 du palier 50 d'une part et cette couronne intérieure de la pièce intermédiaire 34 dans laquelle est justement logé le palier 50.

La figure 11 représente schématiquement une vue globale en perspective coupée d'un exemple d'ensemble tournant de l'organe mécanique du réservoir de liquide biopharmaceutique selon un premier mode de réalisation de l'invention.

Dans une troisième étape de montage de l'organe mécanique 4, l'arbre de rotation 31 portant le chapeau 32 rentre dans la partie haute de la pièce intermédiaire de liaison 34 de manière à se clipser dedans, par des ergots radiaux externes situés en partie basse de l'arbre de rotation 31, coopérant avec des creux situés en partie médiane de la pièce intermédiaire de liaison 34.

La figure 12 représente schématiquement une vue locale en perspective d'un exemple d'une partie d'ensemble tournant de l'organe mécanique du réservoir de liquide biopharmaceutique selon un premier mode de réalisation de l'invention.

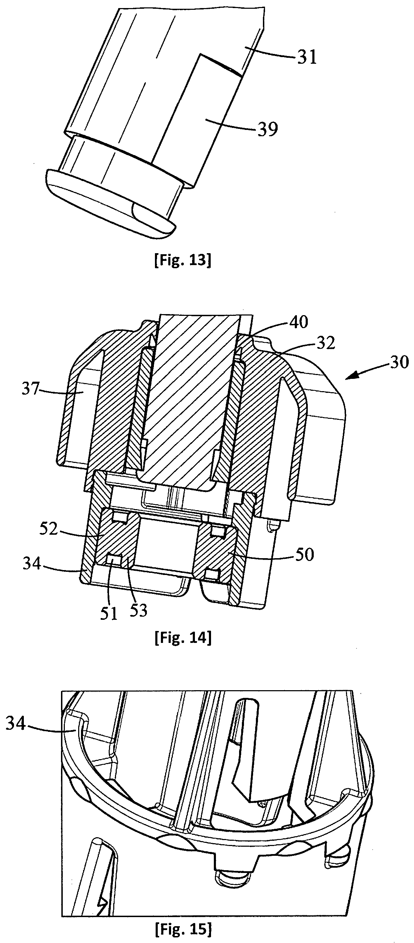

La figure 13 représente schématiquement une vue locale en perspective d'un exemple d'une partie d'ensemble tournant de l'organe mécanique du réservoir de liquide biopharmaceutique selon un premier mode de réalisation de l'invention.

La pièce intermédiaire de liaison 34 présente un méplat 38 qui va coopérer avec le méplat 39 de l'arbre de rotation 31, pour bloquer la rotation relative de l'arbre de rotation 31 par rapport à la pièce intermédiaire de liaison 34.

La couronne intérieure haute de la pièce intermédiaire de liaison 34 comprend sur sa surface interne quatre protubérances disposées pour absorber les tolérances de fabrication et éviter une rotation libre entre l'arbre de rotation 31 d'une part et cette couronne intérieure de la pièce intermédiaire 34 dans laquelle est justement logé l'arbre de rotation 31.

La figure 14représente schématiquement une vue globale en perspective coupée d'un exemple d'ensemble tournant de l'organe mécanique du réservoir de liquide biopharmaceutique selon un premier mode de réalisation de l'invention.

Dans une quatrième étape de montage de l'organe mécanique 4, le chapeau 32, situé autour de l'arbre de rotation 31, coulisse autour de l'arbre de rotation 31 en descendant jusqu'à buter contre le décrochement de la pièce intermédiaire de liaison 34, ce décrochement étant situé à la jonction entre la couronne haute et la couronne basse de cette pièce intermédiaire de liaison 34.

La figure 15 représente schématiquement une vue locale en perspective d'un exemple d'une partie d'ensemble tournant de l'organe mécanique du réservoir de liquide biopharmaceutique selon un premier mode de réalisation de l'invention.

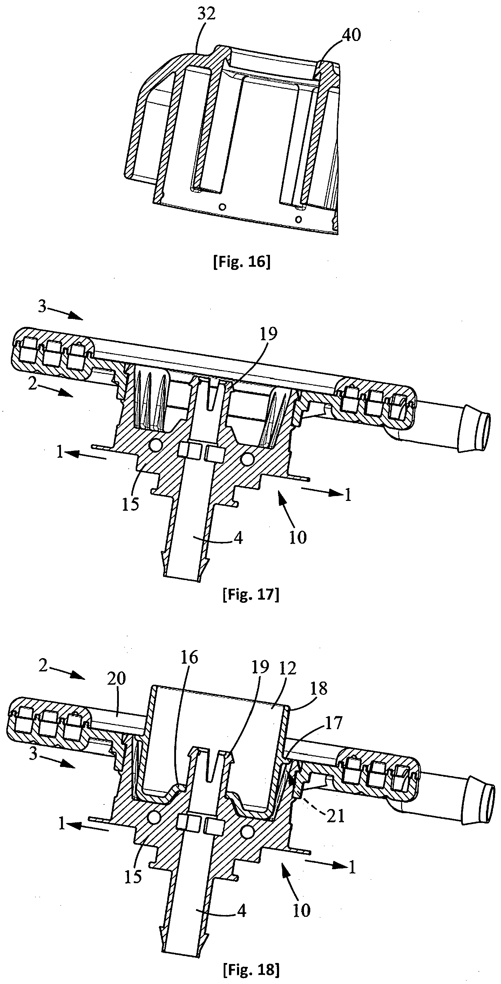

La figure 16 représente schématiquement une vue locale en perspective d'un exemple d'une partie d'ensemble tournant de l'organe mécanique du réservoir de liquide biopharmaceutique selon un premier mode de réalisation de l'invention.

Sur la couronne basse extérieure de la pièce intermédiaire de liaison 34 sont réparties huit petits creux coopérant avec les huit petites protubérances réparties sur la surface interne basse du chapeau 32, pour clipser le chapeau 32 sur l'extérieur de la pièce intermédiaire de liaison 34.

La figure 17 représente schématiquement une vue globale en perspective coupée d'un exemple d'ensemble fixe de l'organe mécanique du réservoir de liquide biopharmaceutique selon un premier mode de réalisation de l'invention.

Dans une cinquième étape de montage de l'organe mécanique 4, le distributeur 20 de gaz est clipsé autour du socle 15 grâce aux ergots radiaux externes du socle 15 situés en partie haute de la périphérie du socle 15.

La figure 18 représente schématiquement une vue globale en perspective coupée d'un exemple d'ensemble fixe de l'organe mécanique du réservoir de liquide biopharmaceutique selon un premier mode de réalisation de l'invention.

Dans une sixième étape de montage de l'organe mécanique 4, la cuvette 1 est rentrée jusqu'au fond du réceptacle du socle 15. La gouttière 17 située en partie médiane externe de la cuvette 12 pousse les ergots radiaux externes supérieurs du socle 15, contribuant ainsi à bien maintenir le clipsage du distributeur 20 de gaz autour de la périphérie externe du socle 15.

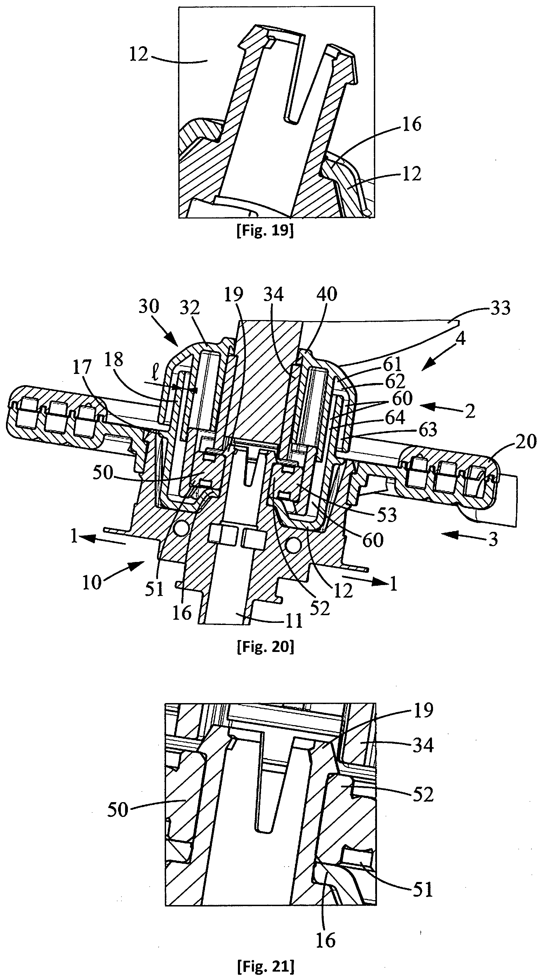

La figure 19 représente schématiquement une vue locale en perspective coupée d'un exemple d'une partie d'ensemble fixe de l'organe mécanique du réservoir de liquide biopharmaceutique selon un premier mode de réalisation de l'invention.

La lèvre intérieure 16 assure d'une part le maintien de la cuvette 12 dans le fond du réceptacle du socle 15 jusqu'à l'étape de montage suivante, puis une fois le montage terminé, contribuera à assurer l'étanchéité entre la cuvette 12 et le socle 15.

La figure 20 représente schématiquement une vue globale en perspective coupée d'un exemple d'ensemble fixe de l'organe mécanique du réservoir de liquide biopharmaceutique selon un premier mode de réalisation de l'invention.

Dans une septième étape de montage de l'organe mécanique 4, le palier 50 est clipsé autour de la cheminée 19 du socle 15, grâce aux ergots radiaux externes de la partie haute de cette cheminée 19.

La figure 21 représente schématiquement une vue locale en perspective coupée d'un exemple d'une partie d'ensemble fixe de l'organe mécanique du réservoir de liquide biopharmaceutique selon un premier mode de réalisation de l'invention.

Le clipsage du palier 50 autour de la cheminée 19 sera encore plus aisément réalisé en utilisant du polyéthylène basse densité (LLDPE) pour la cheminée 19, au moins partiellement.

La figure 22 représente schématiquement une vue globale en perspective coupée d'un exemple d'ensemble fixe de l'organe mécanique du réservoir de liquide biopharmaceutique selon un premier mode de réalisation de l'invention.

Dans une huitième étape de montage de l'organe mécanique 4, la pièce de fixation 14 est introduite par le port de vidange 11 jusqu'à arriver dans la cheminée 19. Cette pièce de fixation 14 sera préférentiellement emmanchée dans le socle 15 avec un outil manuel ayant une position d'arrêt pour être sûr d'avoir bien emmanché cette pièce de liaison 14 jusqu'en haut de la cheminée 19.

La figure 23 représente schématiquement une vue globale en perspective coupée d'un exemple d'assemblage des ensembles fixe et tournant de l'organe mécanique du réservoir de liquide biopharmaceutique selon un premier mode de réalisation de l'invention, montrant la dérivation reliant le distributeur de gaz et le passage de communication.

Une dérivation 21 relie l'intérieur du distributeur 20 de gaz à l'intérieur du passage de communication 60. La fonction de cette dérivation 21 est de pouvoir souffler de l'air dans le passage de communication 60 de manière à tendre à le faire ressortir dans l'espace intérieur 2 de stockage. Dans la réalisation préférentielle de la figure 23, ce canal de dérivation 21, d'une part débouche à l'une de ses extrémités dans l'anneau formant l'intérieur du distributeur 20 de gaz, et plus précisément dans sa paroi intérieure, et d'autre part débouche à l'autre de ses extrémités dans la branche 64 partant du fond 62 du siphon inversé 61 et allant jusqu'au palier 50, et plus précisément dans sa paroi extérieure. Les parois intérieure et extérieure sont définies par rapport au centre de symétrie du réservoir, la paroi intérieure d'un élément donné étant radialement plus proche de ce centre de symétrie que la paroi extérieure de cet élément.

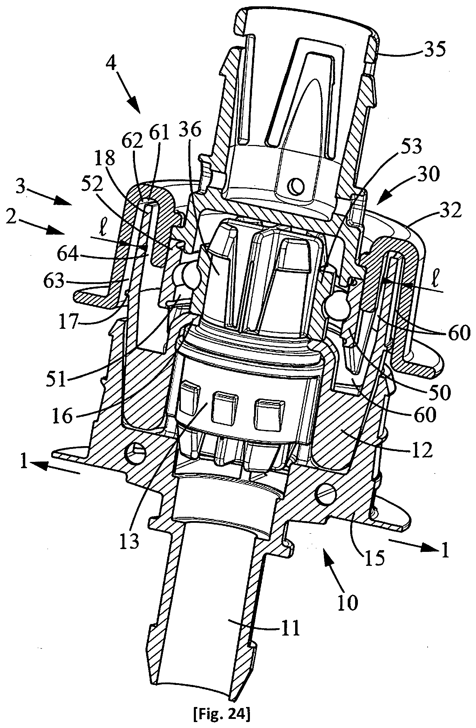

La figure 24 représente schématiquement une vue globale en perspective coupée d'un exemple d'assemblage des ensembles fixe et tournant de l'organe mécanique du réservoir de liquide biopharmaceutique selon un deuxième mode de réalisation de l'invention. Le deuxième mode de réalisation est similaire au premier mode de réalisation, sauf en certains aspects mentionnés dans la suite du texte.

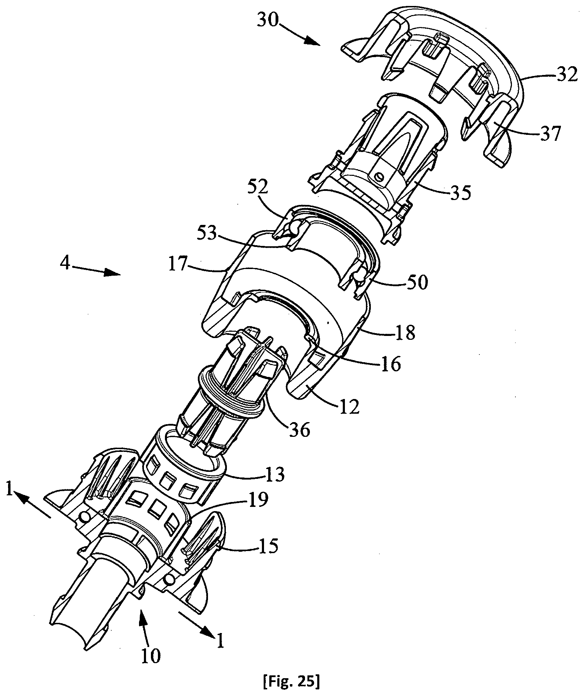

La figure 25 représente schématiquement une vue globale éclatée en perspective coupée d'un exemple d'assemblage des ensembles fixe et tournant de l'organe mécanique du réservoir de liquide biopharmaceutique selon un deuxième mode de réalisation de l'invention.

La pièce intermédiaire de liaison 34 et la pièce de fixation 14 ont été remplacées par un tronc central 36 dont la partie haute est entourée par la bague intérieure 53 du palier 50 et recouverte par une pièce supérieure 35 reposant sur le haut de la bague extérieure 52 du palier 50, la partie basse du tronc central 36 étant entourée par une bague inférieure 13. Le tronc central 36 et la bague inférieure 13 appartiennent à l'ensemble fixe 10, tandis que la pièce supérieure 35 appartient à l'ensemble tournant 30. La bague extérieure 52 du palier 50 reste solidarisée à l'ensemble tournant 30, tandis que la bague intérieure 53 du palier 50 reste solidarisée à l'ensemble fixe 10. La pièce supérieure 35 présente des trous pour limiter les zones mortes. Dans l'ensemble fixe 10, du centre vers la périphérie, le tronc central 36 pousse radialement sur la bague inférieure 13 qui pousse radialement sur la cheminée 19 du socle 15, laquelle cheminée (visible seulement sur la figure 25 mais pas sur la figure 24) pousse radialement sur la cuvette 12 qui pousse radialement sur la périphérie du socle 15.

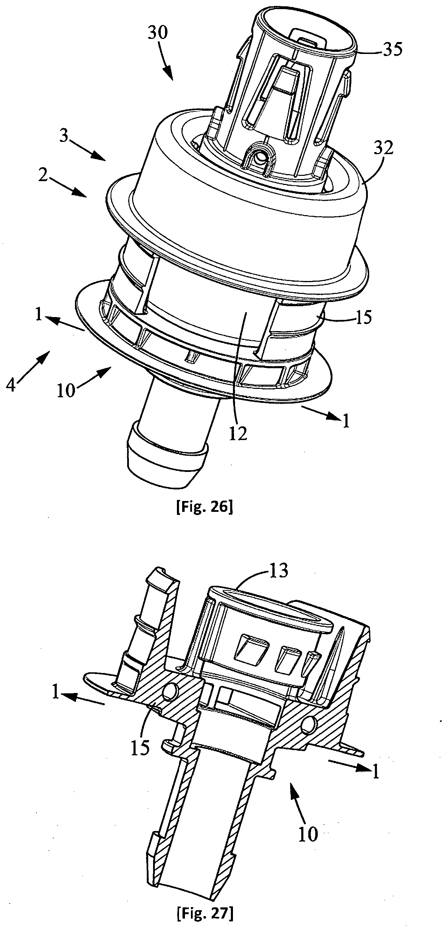

La figure 26 représente schématiquement une vue globale en perspective d'un exemple d'assemblage des ensembles fixe et tournant de l'organe mécanique du réservoir de liquide biopharmaceutique selon un deuxième mode de réalisation de l'invention. L'organe mécanique 4 est représenté en position complètement montée.

La figure 27 représente schématiquement une vue globale en perspective coupée d'un exemple d'ensemble fixe de l'organe mécanique du réservoir de liquide biopharmaceutique selon un deuxième mode de réalisation de l'invention.

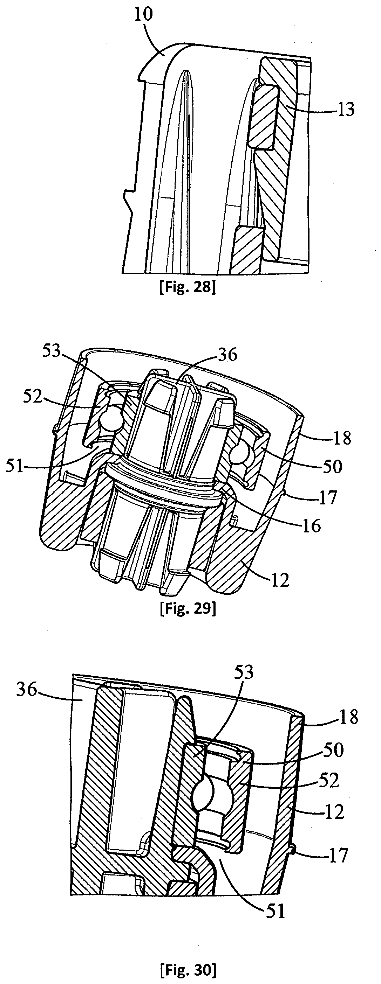

La figure 28 représente schématiquement une vue locale en perspective coupée d'un exemple d'une partie d'ensemble fixe de l'organe mécanique du réservoir de liquide biopharmaceutique selon un deuxième mode de réalisation de l'invention.

Dans une première étape de montage de l'organe mécanique 4, la bague inférieure 13 est clipsée à l'intérieur de la cheminée 19 centrale du socle 15. Des protubérances périphériques inclinées de manière conique dans le sens de la pénétration et disposées sur la surface externe de la bague inférieure 13 viennent se clipser dans des trous correspondants de la cheminée centrale 19 du socle 15.

La figure 29 représente schématiquement une vue globale en perspective coupée d'un exemple d'ensemble tournant de l'organe mécanique du réservoir de liquide biopharmaceutique selon un deuxième mode de réalisation de l'invention.

La figure 30 représente schématiquement une vue locale en perspective coupée d'un exemple d'une partie d'ensemble tournant de l'organe mécanique du réservoir de liquide biopharmaceutique selon un deuxième mode de réalisation de l'invention.

Dans une deuxième étape de montage de l'organe mécanique 4, le palier 50 est clipsé autour du tronc central 36, et plus précisément autour de pattes externes ayant des ergots radiaux externes, la bague intérieure 53 du palier 50 étant bloquée entre ces ergots radiaux externes de ces pattes externes du tronc central 36 et la lèvre intérieure 16 de la cuvette 12. La partie basse du tronc central 36 comprend quatre protubérances périphériques de manière à éviter une rotation libre entre le tronc central 36 et la bague inférieure 13.

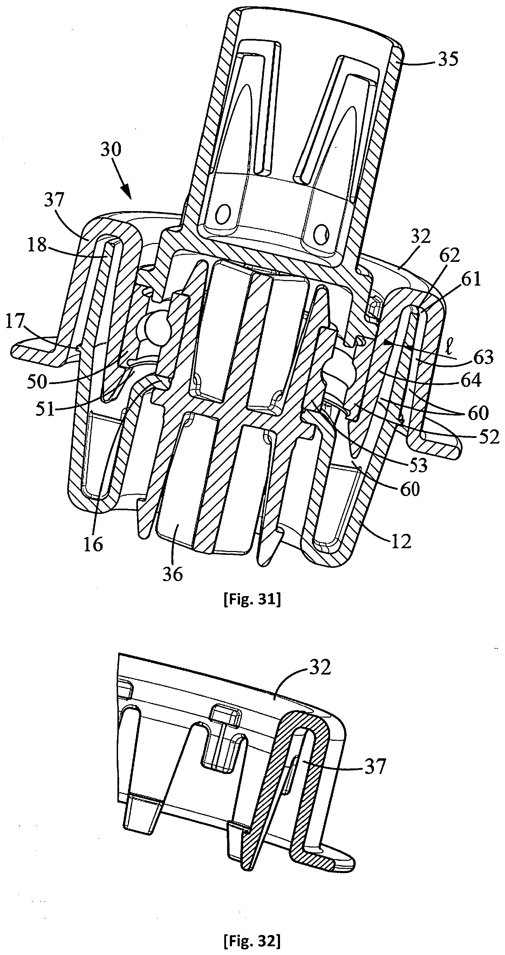

La figure 31 représente schématiquement une vue globale en perspective coupée d'un exemple d'ensemble tournant de l'organe mécanique du réservoir de liquide biopharmaceutique selon un deuxième mode de réalisation de l'invention.

La figure 32 représente schématiquement une vue locale en perspective coupée d'un exemple d'une partie d'ensemble tournant de l'organe mécanique du réservoir de liquide biopharmaceutique selon un deuxième mode de réalisation de l'invention.

Dans une troisième étape de montage de l'organe mécanique 4, la pièce supérieure 35 est clipsée, par sa protubérance annulaire en partie basse, dans la paroi interne du chapeau 32 laquelle comprend huit petites protubérances pour absorber, les tolérances de fabrication et empêcher une rotation libre dans le chapeau 32, à la fois de la pièce supérieure 35 et de la bague extérieure 52 du palier 50.

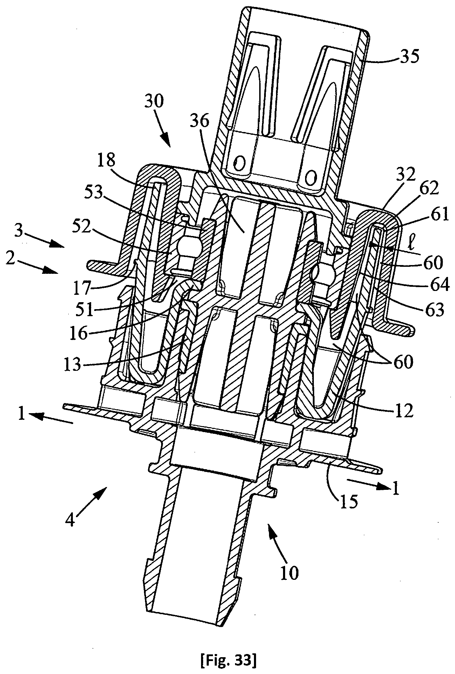

La figure 33 représente schématiquement une vue globale en perspective coupée d'un exemple d'ensemble fixe de l'organe mécanique du réservoir de liquide biopharmaceutique selon un deuxième mode de réalisation de l'invention.

Dans une quatrième étape de montage de l'organe mécanique 4, l'arbre de rotation (non représenté sur la figure 33) est emmanché dans la pièce supérieure 35.

Dans une cinquième étape de montage de l'organe mécanique 4, le distributeur de gaz (non représenté sur la figure 33) est clipsé autour du reste de l'organe mécanique 4.

Dans une sixième étape de montage de l'organe mécanique 4, l'organe mécanique 4 est fixé à l'intérieur de l'ouverture de la paroi de la poche 1.

## Revendications

1. Réservoir de liquide biopharmaceutique, comprenant :
> une poche (1) formant un espace intérieur (2) de stockage du liquide biopharmaceutique (3),
> un organe mécanique (4) situé au niveau d'une paroi de la poche (1), comprenant :
- un ensemble de pièces, fixe (10) par rapport à la paroi de la poche (1),
- un ensemble de pièces, tournant (30) autour d'un axe de rotation (31) par rapport à l'ensemble fixe (10),
- un palier (50) situé entre les deux ensembles (10, 30) et à l'intérieur (2) de la poche (1),
- un passage de communication (60) séparant le palier (50) de l'espace intérieur (2) de stockage, comprenant un ou plusieurs changements de direction (62), **caractérisé en ce que** le passage de communication est formé par une partie (32) des pièces de l'ensemble tournant (30) située en regard d'une partie (12) des pièces de l'ensemble fixe (10).

2. Réservoir de liquide biopharmaceutique selon la revendication 1, **caractérisé en ce que** le passage (60) forme un siphon inversé (61), de l'air étant piégé dans le fond (62) du siphon inversé (61) par du liquide situé au moins dans une branche (63) du siphon inversé (61) partant du fond (62) du siphon inversé (61) et débouchant dans l'espace intérieur (2) de stockage, de préférence ledit air étant piégé dans le fond (62) du siphon inversé (61) par du liquide situé au moins dans les deux branches (63, 64) du siphon inversé (61), la branche (63) partant du fond (62) du siphon inversé (61) et débouchant dans l'espace intérieur (2) de stockage et la branche (64) partant du fond (62) du siphon inversé (61) et allant jusqu'au palier (50).

3. Réservoir de liquide biopharmaceutique selon l'une quelconque des revendications précédentes, **caractérisé en ce que** le passage de communication (60) chemine majoritairement entre deux pièces (12, 32) qui sont d'une part un chapeau (32) de l'ensemble tournant (30) et une cuvette (12) de l'ensemble fixe (10), le chapeau (32) tournant autour de l'axe de rotation (31) par rapport à la cuvette (12), le chapeau (32) et la cuvette (12) étant imbriquées au moins partiellement l'un dans l'autre pour former le passage de communication (60).

4. Réservoir de liquide biopharmaceutique selon l'une quelconque des revendications précédentes, **caractérisé en ce que** l'un des ensembles (10, 30) forme une protubérance (18) rentrant dans un creux (37) de l'autre ensemble (30), le passage de communication (60) contournant la protubérance (18) en cheminant le long des parois du creux (37), et, de préférence, l'ensemble tournant (30) forme le creux (37) et l'ensemble fixe (10) forme la protubérance (18).

5. Réservoir de liquide biopharmaceutique selon l'une quelconque des revendications précédentes, **caractérisé en ce que** le passage de communication (60) est un passage présentant une symétrie de révolution autour de l'axe de rotation (31), de préférence la largeur (l) du passage de communication (60) dans un plan contenant l'axe de rotation (31) est comprise entre 0.5mm et 5mm, de préférence compris entre 1mm et 3mm, de préférence encore le passage de communication (60) comprend au moins deux couloirs parallèles (63, 64) à l'axe de rotation (31), séparés par un virage (62), et, de préférence encore, l'un (64) des couloirs parallèles (63, 64) à l'axe de rotation (31) relie le virage (62) à une cavité (51) comprenant le palier (50).

6. Réservoir de liquide biopharmaceutique selon l'une quelconque des revendications précédentes, **caractérisé en ce que** le passage de communication (60) comprend des ailettes radiales (113 à 117), avantageusement régulièrement réparties autour de l'axe de rotation (31), avantageusement situées dans la branche (63) du siphon inversé (61) partant du fond (62) du siphon inversé (61) et débouchant dans l'espace intérieur (2) de stockage, avantageusement au nombre de 8 à 15 ailettes, de préférence les ailettes radiales (115 à 117) s'étendent à partir de la face externe (112) de la paroi interne (112) d'une partie (18) de l'ensemble fixe (10), et, de préférence encore, les ailettes radiales (117) s'étendent de manière inclinée par rapport à une paroi (111, 112) de l'intérieur du passage de communication (60), le sens de l'inclinaison étant tel que compte-tenu du sens de rotation de l'ensemble tournant (30) autour de l'ensemble fixe (10) d'éventuelles particules solides situées dans le passage de communication (60) restent retenues ou bloquées entre les ailettes (117) et ladite paroi (111, 112) de l'intérieur du passage de communication (60).

7. Réservoir de liquide biopharmaceutique selon l'une quelconque des revendications précédentes, **caractérisé en ce que** le palier (50) est la seule zone de support de l'ensemble tournant (30) par l'ensemble fixe (10), de préférence le palier (50) est la seule zone de contact entre l'ensemble tournant (30) et l'ensemble fixe (10), et, de préférence encore, le palier (50) est un roulement à billes.

8. Réservoir de liquide biopharmaceutique selon l'une quelconque des revendications précédentes, **caractérisé en ce que** l'organe mécanique (4) traverse la paroi de la poche (1) au niveau d'une ouverture de la paroi de la poche (1), de préférence l'ouverture de la paroi de la poche (1) est fixée de manière étanche autour de l'organe mécanique (4), et, de préférence encore, l'ouverture est soudée ou collée autour de l'ensemble fixe (10).

9. Réservoir de liquide biopharmaceutique selon l'une quelconque des revendications précédentes, **caractérisé en ce que** l'ensemble tournant (30) porte un arbre de rotation (31), de préférence l'arbre de rotation (31) porte lui-même une hélice (33).

10. Réservoir de liquide biopharmaceutique selon l'une quelconque des revendications précédentes, **caractérisé en ce que** l'ensemble fixe (10) comprend un port de vidange débouchant à l'extérieur de la poche (1).

11. Réservoir de liquide biopharmaceutique selon l'une quelconque des revendications précédentes, **caractérisé en ce qu'**un distributeur (20) de gaz est situé autour des ensembles (10, 30), dans l'espace intérieur (2) de stockage, de préférence le distributeur (20) de gaz est annulaire, et, de préférence encore, le distributeur (20) de gaz comprend au moins une dérivation (21) apte à souffler de l'air dans le passage de communication (60) de manière à tendre à le faire ressortir dans l'espace intérieur (2) de stockage.

12. Réservoir de liquide biopharmaceutique selon l'une quelconque des revendications précédentes, **caractérisé en ce que** l'ensemble tournant (30) comprend une pièce intermédiaire de liaison (34) portant l'arbre de rotation (31) et le palier (50), de préférence l'arbre de rotation (31) est clipsé dans la pièce intermédiaire de liaison (34), de préférence encore le palier (50) comprend une bague tournante (52), de préférence la bague extérieure (52) du palier (50), qui est clipsée dans la pièce intermédiaire de liaison (34), et, de préférence encore, la pièce intermédiaire de liaison (34) porte le chapeau (32) tournant qui est clipsé sur cette pièce intermédiaire de liaison (34).

13. Réservoir de liquide biopharmaceutique selon l'une quelconque des revendications précédentes, **caractérisé en ce que** l'ensemble fixe (10) comprend un socle (15) qui porte le palier (50) et qui présente un port de vidange (11) débouchant à l'extérieur de l'espace intérieur (2) de stockage, de préférence le distributeur (20) de gaz est clipsé autour du socle (15), de préférence encore le palier (50) est clipsé autour du socle (15), et, de préférence encore, le palier (50) comprend une bague fixe (53), qui est de préférence la bague intérieure (53) du palier (50), qui maintient la cuvette (12) bloquée contre le socle (15).

14. Réservoir de liquide biopharmaceutique selon l'une quelconque des revendications précédentes, **caractérisé en ce que** le liquide biopharmaceutique (3) comprend des micro-supports inertes, dont la plus grande dimension est inférieure à 0.3mm, de préférence inférieure à 0.1mm, avantageusement supérieure à 10µm, des cellules accrochées à ces micro-supports, de préférence les micro-supports sont des billes.

15. Réservoir de liquide biopharmaceutique selon l'une quelconque des revendications précédentes, **caractérisé en ce que** la majorité des pièces des ensembles (10, 30) sont en plastique rigide, légèrement déformables de manière à pouvoir réaliser une opération de clipsage avec elles, sont de préférence en polyéthylène (PE), de préférence la paroi de la poche (1) est en matériau suffisamment souple et déformable pour pouvoir être pliée.

## Patentansprüche

1. Biopharmazeutischer Flüssigkeitsbehälter, umfassend:
> einen Beutel (1), der einen inneren Raum (2) zur Lagerung der biopharmazeutischen Flüssigkeit (3) bildet,
> ein mechanisches Organ (4), das sich auf Höhe einer Wand des Beutels (1) befindet, umfassend:
- einen Satz von Teilen, fest (10) in Bezug auf die Wand des Beutels (1),
- einen Satz von Teilen, drehend (30) um eine Drehachse (31) in Bezug auf den festen Satz (10),
- ein Lager (50), das sich einerseits zwischen den beiden Sätzen (10, 30) und andererseits im Inneren (2) des Beutels (1) befindet,
- einen Kommunikationsdurchgang (60), der das Lager (50) vom inneren Lagerraum (2) trennt, umfassend eine oder mehrere Richtungsänderungen (62),
**dadurch gekennzeichnet, dass** der Kommunikationsdurchgang durch einen Abschnitt (32) der Teile des drehenden Satzes (30) gebildet wird, der sich gegenüber einem Abschnitt (12) der Teile des festen Satzes (10) befindet.

2. Biopharmazeutischer Flüssigkeitsbehälter nach Anspruch 1, **dadurch gekennzeichnet, dass** der Durchgang (60) einen umgekehrten Siphon (61) bildet, wobei Luft im Boden (62) des umgekehrten Siphons (61) durch Flüssigkeit eingeschlossen wird, die sich mindestens in einem Zweig (63) des umgekehrten Siphons (61) befindet, ausgehend vom Boden (62) des umgekehrten Siphons (61) und in den inneren Lagerraum (2) mündend, wobei vorzugsweise die besagte Luft im Boden (62) des umgekehrten Siphons (61) durch Flüssigkeit eingeschlossen wird, die sich mindestens in den beiden Zweigen (63, 64) des umgekehrten Siphons (61) befindet, wobei der Zweig (63) vom Boden (62) des umgekehrten Siphons (61) ausgeht und in den inneren Lagerraum (2) mündet und der Zweig (64) vom Boden (62) des umgekehrten Siphons (61) ausgeht und bis zum Lager (50) verläuft.

3. Biopharmazeutischer Flüssigkeitsbehälter nach einem der vorhergehenden Ansprüche, **dadurch gekennzeichnet, dass** der Kommunikationsdurchgang (60) überwiegend zwischen zwei Teilen (12, 32) verläuft, die eine Kappe (32) des drehenden Satzes (30) und eine Schale (12) des festen Satzes (10) sind, wobei die Kappe (32) sich um die Drehachse (31) in Bezug auf die Schale (12) dreht, wobei die Kappe (32) und die Schale (12) mindestens teilweise ineinander verschachtelt sind, um den Kommunikationsdurchgang (60) zu bilden.

4. Biopharmazeutischer Flüssigkeitsbehälter nach einem der vorhergehenden Ansprüche, **dadurch gekennzeichnet, dass** einer der Sätze (10, 30) einen Vorsprung (18) bildet, der in eine Vertiefung (37) des anderen Satzes (30) eingreift, wobei der Kommunikationsdurchgang (60) den Vorsprung (18) entlang der Wände der Vertiefung (37) umgibt, und wobei vorzugsweise der drehende Satz (30) die Vertiefung (37) bildet und der feste Satz (10) den Vorsprung (18) bildet.

5. Biopharmazeutischer Flüssigkeitsbehälter nach einem der vorhergehenden Ansprüche, **dadurch gekennzeichnet, dass** der Kommunikationsdurchgang (60) ein Durchgang ist, der eine Rotationssymmetrie um die Drehachse (31) aufweist, wobei vorzugsweise die Breite (1) des Kommunikationsdurchgangs (60) in einer Ebene, die die Drehachse (31) enthält, zwischen 0,5 mm und 5 mm liegt, vorzugsweise zwischen 1 mm und 3 mm, wobei ferner vorzugsweise der Kommunikationsdurchgang (60) mindestens zwei parallele Kanäle (63, 64) parallel zur Drehachse (31) umfasst, die durch eine Biegung (62) getrennt sind, und wobei ferner vorzugsweise einer (64) der parallelen Kanäle (63, 64) parallel zur Drehachse (31) die Biegung (62) mit einem Hohlraum (51) verbindet, der das Lager (50) umfasst.

6. Biopharmazeutischer Flüssigkeitsbehälter nach einem der vorhergehenden Ansprüche, **dadurch gekennzeichnet, dass** der Kommunikationsdurchgang (60) radiale Rippen (113 bis 117) umfasst, die vorteilhafterweise regelmäßig um die Drehachse (31) verteilt sind, die sich vorteilhafterweise in dem Zweig (63) des umgekehrten Siphons (61) befinden, der vom Boden (62) des umgekehrten Siphons (61) ausgeht und in den inneren Lagerraum (2) mündet, vorteilhafterweise in einer Anzahl von 8 bis 15 Rippen, wobei vorzugsweise die radialen Rippen (115 bis 117) sich von der Außenfläche (112) eines inneren Teils (112) eines Abschnitts (18) des festen Satzes (10) erstrecken, und wobei ferner vorzugsweise die radialen Rippen (117) sich geneigt in Bezug auf eine Wand (111, 112) des Inneren des Kommunikationsdurchgangs (60) erstrecken, wobei die Richtung der Neigung so ist, dass unter Berücksichtigung der Drehrichtung des drehenden Satzes (30) um den festen Satz (10) etwaige feste Partikel, die sich im Kommunikationsdurchgang (60) befinden, zwischen den Rippen (117) und der besagten Wand (111, 112) des Inneren des Kommunikationsdurchgangs (60) zurückgehalten oder blockiert bleiben.

7. Biopharmazeutischer Flüssigkeitsbehälter nach einem der vorhergehenden Ansprüche, **dadurch gekennzeichnet, dass** das Lager (50) die einzige Stützzone des drehenden Satzes (30) durch den festen Satz (10) ist, wobei vorzugsweise das Lager (50) die einzige Kontaktzone zwischen dem drehenden Satz (30) und dem festen Satz (10) ist, und wobei ferner vorzugsweise das Lager (50) ein Kugellager ist.

8. Biopharmazeutischer Flüssigkeitsbehälter nach einem der vorhergehenden Ansprüche, **dadurch gekennzeichnet, dass** das mechanische Organ (4) die Wand des Beutels (1) auf Höhe einer Öffnung der Wand des Beutels (1) durchquert, wobei vorzugsweise die Öffnung der Wand des Beutels (1) dicht um das mechanische Organ (4) herum befestigt ist, und wobei ferner vorzugsweise die Öffnung um den festen Satz (10) herum geschweißt oder geklebt ist.

9. Biopharmazeutischer Flüssigkeitsbehälter nach einem der vorhergehenden Ansprüche, **dadurch gekennzeichnet, dass** der drehende Satz (30) eine Drehwelle (31) trägt, wobei vorzugsweise die Drehwelle (31) selbst einen Propeller (33) trägt.

10. Biopharmazeutischer Flüssigkeitsbehälter nach einem der vorhergehenden Ansprüche, **dadurch gekennzeichnet, dass** der feste Satz (10) einen Ablassanschluss umfasst, der zur Außenseite des Beutels (1) mündet.

11. Biopharmazeutischer Flüssigkeitsbehälter nach einem der vorhergehenden Ansprüche, **dadurch gekennzeichnet, dass** ein Gasverteiler (20) um die Sätze (10, 30) herum angeordnet ist, im inneren Lagerraum (2), wobei vorzugsweise der Gasverteiler (20) ringförmig ist, und wobei ferner vorzugsweise der Gasverteiler (20) mindestens eine Ableitung (21) umfasst, die geeignet ist, Luft in den Kommunikationsdurchgang (60) zu blasen, um diese tendenziell in den inneren Lagerraum (2) austreten zu lassen.

12. Biopharmazeutischer Flüssigkeitsbehälter nach einem der vorhergehenden Ansprüche, **dadurch gekennzeichnet, dass** der drehende Satz (30) ein Zwischenverbindungsstück (34) umfasst, das die Drehwelle (31) und das Lager (50) trägt, wobei vorzugsweise die Drehwelle (31) in das Zwischenverbindungsstück (34) eingeclipst ist, wobei ferner vorzugsweise das Lager (50) einen drehenden Ring (52) umfasst, vorzugsweise den äußeren Ring (52) des Lagers (50), der in das Zwischenverbindungsstück (34) eingeclipst ist, und wobei ferner vorzugsweise das Zwischenverbindungsstück (34) die Kappe (32) trägt, die auf das besagte Zwischenverbindungsstück (34) aufgeclipst ist.

13. Biopharmazeutischer Flüssigkeitsbehälter nach einem der vorhergehenden Ansprüche, **dadurch gekennzeichnet, dass** der feste Satz (10) einen Sockel (15) umfasst, der das Lager (50) trägt und der einen Ablassanschluss (11) aufweist, der zur Außenseite des inneren Lagerraums (2) mündet, wobei vorzugsweise der Gasverteiler (20) um den Sockel (15) herum aufgeclipst ist, wobei ferner vorzugsweise das Lager (50) um den Sockel (15) herum aufgeclipst ist, und wobei ferner vorzugsweise das Lager (50) einen festen Ring (53) umfasst, der vorzugsweise der innere Ring (53) des Lagers (50) ist, der die Schale (12) gegen den Sockel (15) hält.

14. Biopharmazeutischer Flüssigkeitsbehälter nach einem der vorhergehenden Ansprüche, **dadurch gekennzeichnet, dass** die biopharmazeutische Flüssigkeit (3) inerte Mikroträger umfasst, deren größte Abmessung kleiner als 0,3 mm ist, vorzugsweise kleiner als 0,1 mm, vorteilhafterweise größer als 10 µm, Zellen, die an diesen Mikroträgern befestigt sind, wobei die Mikroträger vorzugsweise Kugeln sind.

15. Biopharmazeutischer Flüssigkeitsbehälter nach einem der vorhergehenden Ansprüche, **dadurch gekennzeichnet, dass** die Mehrheit der Teile der Sätze (10, 30) aus starrem Kunststoff besteht, leicht verformbar, um eine Clipsoperation mit ihnen durchführen zu können, vorzugsweise aus Polyethylen (PE), wobei vorzugsweise die Wand des Beutels (1) aus einem Material besteht, das ausreichend geschmeidig und verformbar ist, um gefaltet werden zu können.

## Claims

1. Biopharmaceutical liquid reservoir, comprising:
> a bag (1) forming an interior space (2) for storing the biopharmaceutical liquid (3),
> a mechanical member (4) situated at the level of a wall of the bag (1), comprising:
- a set of parts, fixed (10) relative to the wall of the bag (1),
- a set of parts, rotating (30) about an axis of rotation (31) relative to the fixed set (10),
- a bearing (50) situated on the one hand between the two sets (10, 30) and on the other hand inside (2) the bag (1),
- a communication passage (60) separating the bearing (50) from the interior storage space (2), comprising one or more changes of direction (62),
**characterized in that** the communication passage is formed by a portion (32) of the parts of the rotating set (30) situated facing a portion (12) of the parts of the fixed set (10).

2. Biopharmaceutical liquid reservoir according to claim 1, **characterized in that** the passage (60) forms an inverted siphon (61), air being trapped in the bottom (62) of the inverted siphon (61) by liquid situated at least in one branch (63) of the inverted siphon (61) starting from the bottom (62) of the inverted siphon (61) and opening into the interior storage space (2), preferably said air being trapped in the bottom (62) of the inverted siphon (61) by liquid situated at least in the two branches (63, 64) of the inverted siphon (61), the branch (63) starting from the bottom (62) of the inverted siphon (61) and opening into the interior storage space (2) and the branch (64) starting from the bottom (62) of the inverted siphon (61) and extending to the bearing (50).

3. Biopharmaceutical liquid reservoir according to any one of the preceding claims, **characterized in that** the communication passage (60) runs predominantly between two parts (12, 32) which are a cap (32) of the rotating set (30) and a cup (12) of the fixed set (10), the cap (32) rotating about the axis of rotation (31) relative to the cup (12), the cap (32) and the cup (12) being at least partially nested one within the other to form the communication passage (60).

4. Biopharmaceutical liquid reservoir according to any one of the preceding claims, **characterized in that** one of the sets (10, 30) forms a protuberance (18) entering into a recess (37) of the other set (30), the communication passage (60) running around the protuberance (18) along the walls of the recess (37), and, preferably, the rotating set (30) forms the recess (37) and the fixed set (10) forms the protuberance (18).

5. Biopharmaceutical liquid reservoir according to any one of the preceding claims, **characterized in that** the communication passage (60) is a passage presenting a symmetry of revolution about the axis of rotation (31), preferably the width (l) of the communication passage (60) in a plane containing the axis of rotation (31) is comprised between 0.5mm and 5mm, preferably comprised between 1mm and 3mm, more preferably the communication passage (60) comprises at least two parallel channels (63, 64) parallel to the axis of rotation (31), separated by a bend (62), and, more preferably, one (64) of the parallel channels (63, 64) parallel to the axis of rotation (31) connects the bend (62) to a cavity (51) comprising the bearing (50).

6. Biopharmaceutical liquid reservoir according to any one of the preceding claims, **characterized in that** the communication passage (60) comprises radial fins (113 to 117), advantageously regularly distributed around the axis of rotation (31), advantageously situated in the branch (63) of the inverted siphon (61) starting from the bottom (62) of the inverted siphon (61) and opening into the interior storage space (2), advantageously numbering 8 to 15 fins, preferably the radial fins (115 to 117) extend from the outer face (112) of an inner part (112) of a portion (18) of the fixed set (10), and, more preferably, the radial fins (117) extend in an inclined manner relative to a wall (111, 112) of the interior of the communication passage (60), the direction of the inclination being such that, taking into account the direction of rotation of the rotating set (30) about the fixed set (10), any solid particles situated in the communication passage (60) remain retained or blocked between the fins (117) and said wall (111, 112) of the interior of the communication passage (60).

7. Biopharmaceutical liquid reservoir according to any one of the preceding claims, **characterized in that** the bearing (50) is the only support zone of the rotating set (30) by the fixed set (10), preferably the bearing (50) is the only contact zone between the rotating set (30) and the fixed set (10), and, more preferably, the bearing (50) is a ball bearing.

8. Biopharmaceutical liquid reservoir according to any one of the preceding claims, **characterized in that** the mechanical member (4) passes through the wall of the bag (1) at the level of an opening of the wall of the bag (1), preferably the opening of the wall of the bag (1) is fixed in a sealed manner around the mechanical member (4), and, more preferably, the opening is welded or glued around the fixed set (10).

9. Biopharmaceutical liquid reservoir according to any one of the preceding claims, **characterized in that** the rotating set (30) carries a rotation shaft (31), preferably the rotation shaft (31) itself carries a propeller (33).

10. Biopharmaceutical liquid reservoir according to any one of the preceding claims, **characterized in that** the fixed set (10) comprises a drain port opening to the exterior of the bag (1).

11. Biopharmaceutical liquid reservoir according to any one of the preceding claims, **characterized in that** a gas distributor (20) is situated around the sets (10, 30), in the interior storage space (2), preferably the gas distributor (20) is annular, and, more preferably, the gas distributor (20) comprises at least one bypass (21) adapted to blow air into the communication passage (60) so as to tend to expel it into the interior storage space (2).

12. Biopharmaceutical liquid reservoir according to any one of the preceding claims, **characterized in that** the rotating set (30) comprises an intermediate connecting piece (34) carrying the rotation shaft (31) and the bearing (50), preferably the rotation shaft (31) is clipped into the intermediate connecting piece (34), more preferably the bearing (50) comprises a rotating ring (52), preferably the outer ring (52) of the bearing (50), which is clipped into the intermediate connecting piece (34), and, more preferably, the intermediate connecting piece (34) carries the cap (32) which is clipped onto said intermediate connecting piece (34).

13. Biopharmaceutical liquid reservoir according to any one of the preceding claims, **characterized in that** the fixed set (10) comprises a base (15) which carries the bearing (50) and which has a drain port (11) opening to the exterior of the interior storage space (2), preferably the gas distributor (20) is clipped around the base (15), more preferably the bearing (50) is clipped around the base (15), and, more preferably, the bearing (50) comprises a fixed ring (53), which is preferably the inner ring (53) of the bearing (50), which holds the cup (12) against the base (15).

14. Biopharmaceutical liquid reservoir according to any one of the preceding claims, **characterized in that** the biopharmaceutical liquid (3) comprises inert micro-carriers, whose largest dimension is less than 0.3mm, preferably less than 0.1mm, advantageously greater than 10µm, cells attached to these micro-carriers, preferably the micro-carriers are beads.

15. Biopharmaceutical liquid reservoir according to any one of the preceding claims, **characterized in that** the majority of the parts of the sets (10, 30) are made of rigid plastic, slightly deformable so as to be able to perform a clipping operation therewith, are preferably made of polyethylene (PE), preferably the wall of the bag (1) is made of a material sufficiently flexible and deformable to be able to be folded.
